(19) Europäisches Patentamt
European Patent Office
Office européen des brevets

(11) **EP 4 527 397 A1**

(12) **EUROPEAN PATENT APPLICATION**

(43) Date of publication:
**26.03.2025   Bulletin 2025/13**

(21) Application number: **23382961.3**

(22) Date of filing: **22.09.2023**

(51) International Patent Classification (IPC):
*A61K 35/66* [(2015.01)]   *A61K 35/742* [(2015.01)]
*A61K 35/744* [(2015.01)]   *A61K 35/745* [(2015.01)]
*A61K 35/747* [(2015.01)]   *A61K 36/064* [(2006.01)]
*A61P 3/06* [(2006.01)]

(52) Cooperative Patent Classification (CPC):
(C-Sets available)
**A61K 35/66; A61K 35/742; A61K 35/744;
A61K 35/745; A61K 35/747; A61K 36/064;
A61P 3/06**                                      (Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Igen Biolab Group AG**
**6300 Zug (CH)**

(72) Inventors:
• **MOSCOSO DEL PRADO UCELAY, Juan**
**28760 Tres Cantos (ES)**

• **GUARDIA ALBA, María Jesús**
**18198 HUETOR VEGA (ES)**

(74) Representative: **Padial Martinez, Ana Belen**
**Baylos 5.0 Legal Advisors S.L.**
**Avda. Diagonal 435, 1º 1ª**
**08036 Barcelona (ES)**

Remarks:
The complete document including Reference
Table(s) and the Sequence Listing(s) can be
downloaded from the EPO website

(54) **THERAPEUTIC USE OF A POSTBIOTIC COMPOSITION IN DYSLIPIDEMIAS**

(57)    Postbiotic composition for use in the treatment of dyslipidemia in a subject, comprising microorganisms of the following genera: *Bacillus, Bifidobacterium, Lactobacillus, Saccharomyces* and *Streptococcus,* for topical or oral administration, including a pharmaceutical formulation.

EP 4 527 397 A1

(52) Cooperative Patent Classification (CPC): (Cont.)

C-Sets
**A61K 35/66, A61K 2300/00;**
**A61K 35/742, A61K 2300/00;**
**A61K 35/744, A61K 2300/00;**
**A61K 35/745, A61K 2300/00;**
**A61K 35/747, A61K 2300/00;**
**A61K 36/064, A61K 2300/00**

**Description**

## TECHNICAL FIELD OF THE INVENTION

The present invention describes a postbiotic composition that comprises lysates of probiotic microorganisms for use as a treatment and/or prevention of dyslipidemias.

## BACKGROUND

Hypercholesterolemia is the principal cause of atherosclerosis disease, which is the accumulation of lipid deposits in the arterial blood vessels. These deposits form plaques that may cause lumen occlusion or ruptured haemorrhage. The traditional animal models to study atherosclerosis (murine and human biopsies) have several limitations to properly visualize and identify the triggers of this disease during its early stages. Zebrafish is an emerging animal model to study metabolic alterations relevant to human atherosclerosis. The use of a high cholesterol diet in zebrafish larvae and adults enables the generation of a time and cost-efficient model of hypercholesterolemia, where the major hallmarks of dyslipidemia in humans, such as vascular lesions with accumulation of lipid and cell infiltration, can be reproduced. Therefore, zebrafish has proven to be an excellent animal model to facilitate the identification of novel preventing and therapeutic treatments for hypercholesterolemia.

In recognition of the potential positive effect that certain bacterial strains may have on the animal gut, various strains have been proposed for use in the treatment of a dyslipidemia related disease. The following patent document is cited as example:

Patent document EP3230479B1 discloses probiotic strains of *Lactobacillus reuteri* and *Bifidobacterium breve* that have cholesterol-absorbing capacity. However, this document does not disclose the postbiotic composition of the present invention with the same microorganisms, neither lysates thereof.

Also noteworthy are Spanish patent applications P201930242 and P201930280 by the same inventors of the current postbiotic composition describing a human intestinal microbiome modulating composition obtained from lysates of probiotic microorganisms and its method of obtaining as well as a food supplement comprising the composition, which has utility in the prevention and treatment of disorders caused, or at least promoted, by intestinal disbiosis, those documents do not disclose or suggest the use of a composition obtained from lysates of probiotic microorganisms for the treatment of a dyslipidemia

Accordingly, there is a requirement in the art for new methods of treating dyslipidemias.

Therefore, the present invention aims to solve the problems of the prior art by using an orally or topically administered composition obtained from lysates of probiotic microorganisms for the treatment and/or prevention of dyslipidemias

## DESCRIPTION OF THE INVENTION

In the present report the expressions "composition of the invention" and "postbiotic composition" are synonymous and are used interchangeably.

In the present report, the term microorganisms include both bacteria and yeast.

Immunomodulatory action is understood, in the context of the present invention, as the set of molecules of the cell walls and lysate of cells and non-viable cells that modulate the innate immune response by the release of cytokines and thereby enhances the immune response against a dyslipidemia.

The terms "treatment" and "treating" as used herein refer to the medical management of a subject with the intent to cure, ameliorate, stabilize, or prevent a disease, pathological condition, or disorder. This term includes active treatment, that is, treatment directed specifically toward the improvement of a disease, pathological condition, or disorder, and also includes causal treatment, that is, treatment directed toward removal of the cause of the associated disease, pathological condition, or disorder. In addition, this term includes palliative treatment, that is, treatment designed for the relief of symptoms rather than the curing of the disease, pathological condition, or disorder; preventative treatment, that is, treatment directed to minimizing or partially or completely inhibiting the development of the associated disease, pathological condition, or disorder; and supportive treatment, that is, treatment employed to supplement another specific therapy directed toward the improvement of the associated disease, pathological condition, or disorder. It is understood that treatment, while intended to cure, ameliorate, stabilize, or prevent a disease, pathological condition, or disorder, need not actually result in the cure, amelioration, stabilization or prevention. The effects of treatment can be measured or assessed as described herein and as known in the art as is suitable for the disease, pathological condition, or disorder involved. Such measurements and assessments can be made in qualitative and/or quantitative terms. Thus, for example, characteristics or features of a disease, pathological condition, or disorder and/or symptoms of a disease, pathological condition, or disorder can be reduced to any effect or to any amount. In the context of a subject suffering from a dyslipidemia, the terms "treatment" and "treating" refer to the medical management of a subject with the intent to cure, ameliorate, or stabilize a dyslipidemia.

In the context of a subject at risk of developing a dyslipidemia, the terms "treatment" and "treating" refer to the medical management of a subject with the intent to prevent a dyslipidemia.

The term "preventing" as used herein refers to administering a compound prior to the onset of clinical symptoms of a disease or conditions so as to prevent a physical manifestation of aberrations associated with the disease or condition. In the context of a dyslipidemia, the term "preventing" refers to administering a compound prior to the onset of clinical symptoms of a dyslipidemia so as to prevent a physical manifestation of aberrations associated with a dyslipidemia.

In a first aspect, the present invention relates to a postbiotic composition that comprises lysates of probiotic microorganisms of the genus *Bacillus, Bifidobacterium, Lactobacillus, Saccharomyces* and *Streptococcus* for use in the treatment and/or prevention of a dyslipidemia in a subject.

In a particular embodiment of the invention, the composition comprises lysates of probiotic microorganisms of *Bacillus licheniformis, Bacillus subtilis, Bacillus coagulans, Bifidobacterium bifidum, Bifidobacterium breve, Bifidobacterium lactis, Bifidobacterium animalis susp lactis, Lactobacillus acidophilus, Lactobacillus casei, Lactobacillus plantarum, Lactobacillus reuteri, Lactobacillus rhamnosus, Saccharomyces cerevisiae* and *Streptococcus thermophilus* for use in the treatment and/or prevention of a dyslipidemia in a subject..

In another particular embodiment, the present invention relates to a postbiotic composition that comprises lysates of probiotic microorganisms in a percentage amount by weight with respect to the total weight of the lysates of microorganisms of the composition:

- 0.1% to 10% of bacterial lysates of the genus *Bacillus;*
- 8% to 40% of bacterial lysates of the genus *Bifidobacterium;*
- 8% to 60% of bacterial lysates of the genus *Lactobacillus;*
- 15% to 60% of yeast lysates of the genus *Saccharomyces;*
- 0.5% to 15% of bacterial lysates of the genus *Streptococcus.*

for use in the treatment and/or prevention of a dyslipidemia in a subject.

In another particular embodiment, the present invention relates to a postbiotic composition that comprises lysates of probiotic microorganisms in a percentage amount by weight with respect to the total weight of the lysates of microorganisms of the composition:

- 0.1% to 10% of bacterial lysates of the genus *Bacillus;*
- 8% to 40% of bacterial lysates of the genus *Bifidobacterium;*
- 8% to 40% of bacterial lysates of the genus *Lactobacillus;*
- 20% to 60% of yeast lysates of the genus *Saccharomyces;*
- 0.5% to 15% of bacterial lysates of the genus *Streptococcus.*

for use in the treatment and/or prevention of a dyslipidemia in a subject.

In accordance with the invention, the composition can comprise between 1% - 99.5% by weight of lysates of microorganisms; particularly 5%-99% by weight of lysates of microorganisms, particularly 10%-96% by weight of lysates of microorganisms, particularly 25%-95% by weight of lysates of microorganisms, or particularly 50%±10% by weight of lysates of microorganisms.

In a particular embodiment, the postbiotic composition of the invention is administered orally or topically.

As indicated previously, the inventors have identified the need to develop a composition that is administered both orally and topically, that is easy to administer and that does not present toxicity to the subject.

Based on this, they have developed a postbiotic composition that can be administered orally or topically, which comprises lysates of microorganisms that can be accompanied by other components and which has proven to be surprisingly beneficial for humans and animals, as it has an immunomodulatory character.

The composition of the invention may comprise additional components or additives or any other substance, such as salts or excipients that facilitate the packaging, preparation and/or administration of the composition but do not affect the capacity of treatment the dyslipidemia of the postbiotic composition of the invention.

In the context of the present invention, lysates of probiotic microorganisms are understood to be the product obtained after the procedure of culturing and subsequently mechanically or chemically breaking these cells in order to obtain a product with microbial fragments, as well as all the components contained therein. Likewise, this document states that they are lysates of dried probiotic microorganisms, since, thanks to the method of obtaining them, said lysates are subsequently subjected to drying techniques, in such a way that said dried lysates are optionally in the form of a dry powder.

In preferred embodiments of the present invention, the bacterial lysates of the genus *Bacillus* are of one or more of the species selected from the group consisting of: *Bacillus clausii, Bacillus coagulans, Bacillus licheniformis, Bacillus pumilus, Bacillus subtilis, Bacillus mesentericus, Bacillus paralicheniformes,* and combinations thereof, preferably, *Bacillus licheniformis, Bacillus coagulans* and *Bacillus subtilis.*

In other embodiments, the bacterial lysates of the genus *Bifidobacterium* are of one or more of the species selected from the group consisting of: *Bifidobacterium animalis subsp lactis, Bifidobacterium bifidum, Bifidobacterium breve, Bifidobacterium lactis, Bifidobacterium longum, Bifidobacterium infantis, Bifidobacterium animalis,* and combinations thereof, preferably *Bifidobacterium bifidum, Bifidobacterium breve, Bifidobacterium lactis* and *Bifidobacterium animalis subsp lactis.*

In other embodiments, the bacterial lysates of the genus *Lactobacillus* are of one or more of the species selected from the group consisting of: *Lactobacillus acidophilus, Lactobacillus bulgaricus, Lactobacillus casei, Lactobacillus fermentum, Lactobacillus paracasei, Lactobacillus plantarum, Lactobacillus reuteri, Lactobacillus rhamnosus, Lactobacillus salivarius, Lactobacillus helvéticus, Lactobacillus gasseri, Lactobacillus johnsonii, Lactobacillus brevis, Lactobacillus kefiri,* and combinations thereof, preferably *Lactobacillus acidophilus, Lactobacillus casei, Lactobacillus plantarum, Lactobacillus reuteri* and *Lactobacillus rhamnosus.*

In other particular embodiments of the present invention, the lysates of yeasts of the genus *Saccharomyces* are of the species that are selected from the group consisting of *Saccharomyces cerevisiae, Saccharomyces boulardii,* and combinations thereof, preferably *Saccharomyces cerevisiae.*

In other particular embodiments of the present invention, the bacterial lysates of the genus *Streptococcus* are of the species *Streptococcus thermophilus.*

In preferred embodiments of the present invention, the composition of the invention comprises bacterial lysates of the genus *Bacillus* in an amount in percentage by weight between approximately 0.1 % to 10 % of the total lysates of the composition, preferably 1 % to 8 %, even more preferably between 1.2% to 5% of the total lysates of the composition. In preferred embodiments, the amount of bacterial lysates of the genus *Bacillus* can be approximately 1.2%, 1.4%, 1.6%, 1.8%, 2%, 2.5%, 3%, 3.5%, 4% or 4.5% of the total lysates of the composition.

In a particular embodiment, when the bacterial lysates of the genus *Bacillus* are of the species *Bacillus licheniformis, Bacillus coagulans* and *Bacillus subtilis,* the percentage by weight of *Bacillus licheniformis* and *Bacillus subtilis* is approximately the same, and is higher than that of *Bacillus coagulans.* In another particular embodiment, the percentage by weight of *Bacillus licheniformis, Bacillus coagulans* and *Bacillus subtilis* is approximately the same.

In preferred embodiments of the present invention, the composition of the invention comprises bacterial lysates of the genus *Bifidobacterium* in an amount in percentage by weight between approximately 8% to 40% of the total lysates of the composition, preferably between 12% to 35%, more preferably between 12% to 30%, even more preferably between 20% to 30% of the total lysates of the composition. In preferred embodiments, the amount of bacterial lysates of the genus *Bifidobacterium* can be approximately 13%, 14%, 15%, 16%, 17%, 18%, 19%, 20%, 21%, 22%, 23%, 24%, 25%, 26%, 27%, 28%, or 29% of the total lysates of the composition.

In a particular embodiment, when the bacterial lysates of the genus *Bifidobacterium* are of the species *Bifidobacterium animalis susp lactis, Bifidobacterium breve, Bifidobacterium bifidum* and *Bifidobacterium lactis,* the percentage by weight of *Bifidobacterium animalis susp lactis* and *Bifidobacterium breve* is approximately the same, and is higher than that of *Bifidobacterium bifidum* and *Bifidobacterium lactis.* In another particular embodiment, the percentage by weight of *Bifidobacterium animalis susp lactis* and *Bifidobacterium breve* is approximately the same, and is higher than that of *Bifidobacterium lactis* which is higher than that of *Bifidobacterium bifidum.*

In preferred embodiments of the present invention, the composition of the invention comprises bacterial lysates of the genus *Lactobacillus* in an amount in percentage by weight between approximately 8% to 40% of the total lysates of the composition, preferably between 12% to 35%, more preferably between 12% to 30%, even more preferably between 20% to 30% of the total lysates of the composition. In preferred embodiments, the amount of bacterial lysates of the genus *Lactobacillus* can be approximately 13%, 14%, 15%, 16%, 17%, 18%, 19%, 20%, 21%, 22%, 23%, 24%, 25%, 26%, 27%, 28%, or 29% of the total lysates of the composition.

In a particular embodiment, when the bacterial lysates of the genus *Lactobacillus* are of the species *Lactobacillus acidophilus, Lactobacillus casei, Lactobacillus plantarum, Lactobacillus reuteri* and *Lactobacillus rhamnosus,* the percentage by weight of *Lactobacillus rhamnosus* and *Lactobacillus acidophilus* is approximately the same, and is smaller than *Lactobacillus casei, Lactobacillus plantarum,* and *Lactobacillus reuteri.* In another particular embodiment, the percentage by weight of *Lactobacillus rhamnosus* and *Lactobacillus acidophilus* is approximately the same, and is smaller than *Lactobacillus casei, Lactobacillus plantarum,* and *Lactobacillus reuteri* which is approximately the same.

In preferred embodiments of the present invention, the composition comprises lysates of yeasts of the genus *Saccharomyces* in an amount in percentage by weight of between approximately 20% to 60% of the total lysates of the composition, preferably 25% to 50% of the total lysates of the composition, preferably between 30% to 45% of the total lysates of the composition. In preferred embodiments, the amount of lysates of yeasts of the genus *Saccharomyces* can be approximately 31%, 32%, 33%, 34%, 35%,36%, 37%, 38%, 39%, 40%, 41%, 42%, 43% or 44% of the total lysates of the composition. In a particular embodiment, the bacterial lysates of the genus *Saccharomyces* are of *Saccharomyces*

*cereviseae.*

In preferred embodiments of the present invention, the composition comprises bacterial lysates of the genus *Streptococcus* in an amount in percentage by weight of between about 0.5% to 15% of the total lysates of the composition, preferably between about 1% to 10%, more preferably between about 2% to 7%, even more preferably between about 3.5% to 6% of the total lysates of the composition. In a particular embodiment, the amount of bacterial lysates of the genus *Streptococcus* can be approximately 3.6%, 3.7%, 3.8%, 3.9%, 4.0%, 4.1%, 4.2%, 4.3%, 4.4%, 4.5%, 4.6%, 4.7%, 4.8%, 4.9%, 5.0%, 5.1%, 5.2%, 5.3%, 5.4%, 5.5%, 5.6%, 5.7%, 5.8%, or 5.9% of the total lysates of the composition. In a particular embodiment, the bacterial lysates of the genus *Streptococcus* are of *Streptococcus thermophilus.*

In a particular embodiment of the invention, the composition for use, comprises lysates of probiotic microorganisms in an amount in percentage by weight on the total lysates of the composition:

- approximately 1% to 8% of bacterial lysates of the genus *Bacillus;*
- approximately 12% to 35% of bacterial lysates of the genus *Bifidobacterium;*
- approximately 12% to 35% of bacterial lysates of the genus *Lactobacillus;*
- approximately 25% to 50% of yeast lysates of the genus *Saccharomyces;*
- approximately 1% to 10% of bacterial lysates of the genus *Streptococcus.*

This composition is called composition "A".

In another particular embodiment of the invention, the composition for use, comprises lysates of probiotic microorganisms in an amount in percentage by weight on the total lysates of the composition:

- approximately 1.2% to 5% of bacterial lysates of the genus *Bacillus;*
- approximately 20% to 30% of bacterial lysates of the genus *Bifidobacterium;*
- approximately 20% to 30% of bacterial lysates of the genus *Lactobacillus;*
- approximately 30% to 45% of yeast lysates of the genus *Saccharomyces;*
- approximately 2% to 7% of bacterial lysates of the genus *Streptococcus.*

This composition is called composition "B".

In another particular embodiment of the invention, the composition for use comprises lysates of probiotic microorganisms in an amount in percentage by weight with respect to the total:

- approximately 0.1% to 10% of bacterial lysates of *Bacillus licheniformis, Bacillus coagulans* and *Bacillus subtilis;*
- approximately 8% to 40% of bacterial lysates of *Bifidobacterium animalis susp lactis, Bifidobacterium breve, Bifidobacterium lactis* and *Bifidobacterium bifidum,*
- approximately 8% to 40% of bacterial lysates of *Lactobacillus acidophilus, Lactobacillus casei, Lactobacillus plantarum, Lactobacillus reuteri* and *Lactobacillus rhamnosus;*
- approximately 20% to 60% of yeast lysates of *Saccharomyces cerevisiae;*
- approximately 0.5% to 15% of bacterial lysates of *Streptococcus thermophilus.*

This composition is called composition "C".

In another particular embodiment of the invention, the composition for use comprises lysates of probiotic microorganisms in an amount in percentage by weight with respect to the total:

- approximately 1% to 8% of bacterial lysates of *Bacillus licheniformis, Bacillus coagulans* and *Bacillus subtilis;*
- approximately 12% to 35% of bacterial lysates of *Bifidobacterium animalis susp lactis, Bifidobacterium breve, Bifidobacterium lactis* and *Bifidobacterium bifidum,*
- approximately 12% to 35% of bacterial lysates of *Lactobacillus acidophilus, Lactobacillus casei, Lactobacillus plantarum, Lactobacillus reuteri* and *Lactobacillus rhamnosus;*
- approximately 25% to 50% of yeast lysates of *Saccharomyces cerevisiae;*
- approximately 1% to 10% of bacterial lysates of *Streptococcus thermophilus.*

This composition is called composition "D".

In another particular embodiment of the invention, the composition for use comprises lysates of probiotic microorganisms in an amount in percentage by weight with respect to the total:

- approximately 1.2% to 5% of bacterial lysates of *Bacillus licheniformis, Bacillus coagulans* and *Bacillus subtilis;*
- approximately 20% to 30% of bacterial lysates of *Bifidobacterium animalis susp lactis, Bifidobacterium breve, Bifidobacterium lactis* and *Bifidobacterium bifidum,*

- approximately 20% to 30% of bacterial lysates of *Lactobacillus acidophilus, Lactobacillus casei, Lactobacillus plantarum, Lactobacillus reuteri* and *Lactobacillus rhamnosus;*
- approximately 30% to 45% of yeast lysates of *Saccharomyces cerevisiae;*
- approximately 2% to 7% of bacterial lysates of *Streptococcus thermophilus.*

This composition is called composition "E".

In another particular embodiment of the invention, the composition for use comprises lysates of probiotic microorganisms in an amount in percentage by weight with respect to the total:

- approximately 0.1% to 10% of bacterial lysates of *Bacillus licheniformis, Bacillus coagulans* and *Bacillus subtilis;* being the percentage by weight of *Bacillus licheniformis* and *Bacillus subtilis* approximately the same, and higher than that of *Bacillus coagulans,*
- approximately 8% to 40% of bacterial lysates of *Bifidobacterium animalis susp lactis, Bifidobacterium breve, Bifidobacterium lactis* and *Bifidobacterium bifidum, being* the percentage by weight of *Bifidobacterium animalis susp lactis* and *Bifidobacterium breve* approximately the same, and higher than that of *Bifidobacterium lactis* which is higher than that of *Bifidobacterium bifidum.*
- approximately 8% to 40% of bacterial lysates of *Lactobacillus acidophilus, Lactobacillus casei, Lactobacillus plantarum, Lactobacillus reuteri* and *Lactobacillus rhamnosus* being the percentage by weight of *Lactobacillus rhamnosus* and *Lactobacillus acidophilus* approximately the same, and smaller than that of *Lactobacillus casei, Lactobacillus plantarum,* and *Lactobacillus reuteri* respectively
- approximately 20% to 60% of yeast lysates of *Saccharomyces cerevisiae;*
- approximately 0.5% to 15% of bacterial lysates of *Streptococcus thermophilus.*

This composition is called composition "F".

In another particular embodiment of the invention, the composition for use comprises lysates of probiotic microorganisms in an amount in percentage by weight with respect to the total:

- approximately 1% to 8% of bacterial lysates of *Bacillus licheniformis, Bacillus coagulans* and *Bacillus subtilis;* being the percentage by weight of *Bacillus licheniformis* and *Bacillus subtilis* approximately the same, and higher than that of *Bacillus coagulans,*
- approximately 12% to 35% of bacterial lysates of *Bifidobacterium animalis susp lactis, Bifidobacterium breve, Bifidobacterium lactis* and *Bifidobacterium bifidum, being* the percentage by weight of *Bifidobacterium animalis susp lactis* and *Bifidobacterium breve* approximately the same, and higher than that of *Bifidobacterium lactis* which is higher than that of *Bifidobacterium bifidum.*
- approximately 12% to 35% of bacterial lysates of *Lactobacillus acidophilus, Lactobacillus casei, Lactobacillus plantarum, Lactobacillus reuteri* and *Lactobacillus rhamnosus* being the percentage by weight of *Lactobacillus rhamnosus* and *Lactobacillus acidophilus* approximately the same, and smaller than that of *Lactobacillus casei, Lactobacillus plantarum,* and *Lactobacillus reuteri* respectively
- approximately 25% to 50% of yeast lysates of *Saccharomyces cerevisiae;*
- approximately 1% to 10% of bacterial lysates of *Streptococcus thermophilus.*

This composition is called composition "G".

In another particular embodiment of the invention, the composition for use comprises lysates of probiotic microorganisms in an amount in percentage by weight with respect to the total:

- approximately 1.2% to 5% of bacterial lysates of *Bacillus licheniformis, Bacillus coagulans* and *Bacillus subtilis;* being the percentage by weight of *Bacillus licheniformis* and *Bacillus subtilis* approximately the same, and higher than that of *Bacillus coagulans,*
- approximately 20% to 30% of bacterial lysates of *Bifidobacterium animalis susp lactis, Bifidobacterium breve, Bifidobacterium lactis* and *Bifidobacterium bifidum, being* the percentage by weight of *Bifidobacterium animalis susp lactis* and *Bifidobacterium breve* approximately the same, and higher than that of *Bifidobacterium lactis* which is higher than that of *Bifidobacterium bifidum.*
- approximately 20% to 30% of bacterial lysates of *Lactobacillus acidophilus, Lactobacillus casei, Lactobacillus plantarum, Lactobacillus reuteri* and *Lactobacillus rhamnosus* being the percentage by weight of *Lactobacillus rhamnosus* and *Lactobacillus acidophilus* approximately the same, and smaller than that of *Lactobacillus casei, Lactobacillus plantarum,* and *Lactobacillus reuteri* respectively
- approximately 30% to 45% of yeast lysates of *Saccharomyces cerevisiae;*
- approximately 2% to 7% of bacterial lysates of *Streptococcus thermophilus.*

This composition is called composition "H".

In other particular embodiments of the present invention, additional components or additives may be: carbohydrates (fructose, xylitol, sorbitol, fructooligosaccharides, inulin), antioxidants (resveratrol, beta-carotene), vitamins (Vitamin C, D, K, B1, B9, E, B3, B5, A, B3 , B6, H, D3, B12 biotin, riboflavin, pyridoxine, pantothenic acid), prebiotics (galacto-oligosaccharides, inulin, oligofructose), amino acids (cysteine, tyrosine, resin, methionine, phenylalanine, glycine, glutamine, alanine, carnitine, glutamine, arginine), lipids (eicosapentaenoic acid, docosahexaenoic acid, arachidonic acid), trace elements (sodium, potassium, magnesium, phosphorus, calcium, copper, zinc, manganese, chromium, iodine, selenium), digestive enzymes (papain, amylase, lactase, bromelain), whey.

These micro and macronutrients can modulate the intestinal microbiota and favor the predominance of some species associated with a good state of health, such as species of the genera *Bifidobacterium, Lactobacillus, Akkermansia, Fecalibacterium, Eubacterium, Roseburia, Ruminococcus* and *Blautia.* Among the macronutrients, carbohydrates such as galactooligosaccharides, fructooligosaccharides and inulin have a bifidogenic effect and can modulate microorganisms beneficial to health as well as unsaturated fat, which also increases the beneficial microbiota. Antioxidants such as polyphenols modulate the Firmicutes/Bacteroidetes ratio. Vitamins, such as vitamin A, C, D and E have a positive influence on *Bifidobacteria, Akkermansia* and *Lactobacillus.* Some trace elements such as calcium, magnesium, phosphorus, selenium and zinc have shown an effect on *Akkermansia, Bifidobacterium* and *Ruminococcus.* Prebiotic consumption has been associated with the growth of *Bifidobacterium* and lactic acid bacteria. In general, macro and micronutrients influence the composition and diversity of the gut microbiota.

In a particular embodiment of the invention, the composition of lysates and additional components is that of table 1. Additional components may be one or more of resveratrol, astaxanthin, bromelain, papain, fermented rice starch, corn starch and FOS, each of these components in varying amounts, as shown in Table 1.

Table 1: List of additional components to the composition of the invention per 100 mg of lysates of probiotic microorganisms.

| Composition of lysates of probiotic microorganisms | 100 | Mg |
|---|---|---|
| Resveratrol | Up to 2.55 | Mg |
| Astaxanthin | Up to 25 | Mg |
| Bromelain | Up to 24 | Mg |
| Papain | Up to 35.2 | Mg |
| Fermented rice starch | Up to 110 | Mg |
| Corn starch | Up to 12 | Mg |
| FOS | Up to 70 | Mg |

In the specific example of Table 1, the composition of lysates of probiotic microorganisms would represent 26.4% of the total weight of the composition of the invention.

Another object of the invention relates to a method to obtained the postbiotic composition described above which comprised the following steps:

- Cultivate the microbial species selected for the preparation of the composition using the standard conditions established for the microbial species listed in this document;
- Filter or centrifuge the microbial species until a suitable biomass of the selected microorganisms is obtained;
- Lyse through freezing and thawing and sonication cycles of the biomasses obtained. until securing at least 90% or 91% or 92% or 94% of bacterial lysates, preferably at least 95%, or 96% or 97% or 98% and more preferably, 99% or 100%;
- Mix the different strains in the proportions selected according to the percentage by weight to obtain the composition of lysates of probiotic microorganisms of the invention.

A skilled person would know how to find the appropriate protocol for the growth of the microorganisms of the composition, as well as the protocol for their lysis, since these are common procedures in the prior art.

In a particular embodiment, the method of obtaining the composition of the invention comprises an additional step: drying by atomization or lyophilization the biomasses of bacterial lysates obtaining a product in powder form comprising all the bacterial strains selected to prepare the composition object of the invention.

In a particular embodiment, the composition of the invention is presented in the form of dry powder.

When the composition of the invention is presented in the form of dry powder, it is necessary to dissolve it in water or any other liquid that does not affect the properties of the components of the composition prior to its intake by the patient. The advantages of dry powder are that it is easier to transport and to store, and it also increases the stability and durability of the composition. In the present document, dry powder means that the humidity level is less than 10%, preferably less than 5%, even more preferably less than 1%.

In particular embodiments, the composition of the invention is obtained from cultures of probiotic microorganisms of the genera described in the present document, which comprise a certain amount of Colony Forming Units (CFU). In the context of the present invention, a Colony Forming Unit is a term of microbiology. It is an indicator of the amount of live microorganisms present in a medium.

The process of obtaining lysates comprises a thermal treatment, alternating heating and cooling cycles. Each batch of viable cell culture is resuspended in distilled water in a 1:2 ratio and subjected to a sterilization cycle in an autoclave at 121°C for 20 minutes. They are then frozen at -20°C for 12 hours and thawed at room temperature the next day.

Once thawed, they undergo a cell rupture treatment by sonication for 20min, at an output power of 500 W at an amplitude of 40% and 10 seconds of pause, obtaining a mass of lysed cells from the probiotic batch (Qsonica, Q500). The final solution is freeze-dried and ground to obtain a lysate of the powdery probiotic microorganism. The powder is kept in a cool environment away from heat. The degree of survival in no case exceeds a final viability of approximately 7E+3 CFU/ml, which implies practically 100% cell death.

In particular embodiments of the present invention, one starts with a quantity of bacteria of the genus *Bacillus* comprising approximately between 0.49% to 10.94% of CFU with respect to the total CFU of the composition, preferably between approximately 1.98% and 5.75%. In absolute values, the culture grown, before proceeding to the lysis of the cells, presents between 7E+10 and 5E+11 cells, preferably between 9E+10 and 3E+11, most preferably approximately 1.10E+11.

In another particular embodiment, when the species of the genus *Bacillus* are: *Bacillus licheniformis, Bacillus coagulans* and *Bacillus subtilis,* each of the grown cultures has, before proceeding to the cells lysis, between 1E+10 and 8E+11 of *Bacillus licheniformis* cells, between 1E+10 and 8E+11 of *Bacillus coagulans* cells and between 1E+10 and 8E+11 of *Bacillus subtilis* cells.

In particular embodiments of the present invention, one starts with a quantity of bacteria of the genus *Bifidobacterium* comprising between 5.20% to 33.64% of CFU with respect to the total culture, preferably between 8.99% to 23.75%. In absolute values, the culture grown, before proceeding to the lysis of the cells, presents between 8E+10 and 9E+11 cells, preferably between 1E+11 and 7E+11, most preferably approximately 5.50E+11.

In another particular embodiment, when the species of the genus *Bifidobacterium* are: *Bifidobacterium animalis susp lactis, Bifidobacterium breve, Bifidobacterium lactis* and *Bifidobacterium bifidum,* each of the grown cultures has, before proceeding to the cells lysis, between 8E+10 and 5E+11 of *Bifidobacterium animalis susp lactis* cells, between 8E+10 and 5E+11 of *Bifidobacterium breve* cells, between 8E+10 and 5E+11 of *Bifidobacterium lactis* cells and between 8E+10 and 5E+11 of *Bifidobacterium bifidum* cells.

In particular embodiments of the present invention, one starts with a number of bacteria of the genus *Lactobacillus* comprising between 32.64% to 76.82% of CFU with respect to the total CFU of the composition, preferably between 40.66% to 63.36%. In absolute values, the culture grown, before proceeding to the lysis of the cells, presents between 5E+11 and 5E+12 cells, preferably between 9E+11 and 3E+12, most preferably approximately 1.7E+12.

In another particular embodiment, when the species of the genus *Lactobacillus* are: *Lactobacillus acidophilus, Lactobacillus casei, Lactobacillus plantarum, Lactobacillus reuteri,* and *Lactobacillus rhamnosus,* each of the grown cultures presents, before proceeding to the lysis of the cells, between 8E+10 and 3E+11 cells of *Lactobacillus acidophilus,* between 1E+11 and 7E+11 cells of *Lactobacillus casei,* between 1E+11 and 7E+11 cells of *Lactobacillus plantarum,* between 8E+10 and 3E+11 cells of *Lactobacillus reuteri* and between 1E+11 and 7E+11 cells of *Lactobacillus rhamnosus.*

In particular embodiments of the present invention, one starts with a quantity of bacteria of the genus *Saccharomyces* comprising between 5.44% to 28.79% of CFU with respect to the total culture, preferably between 10.04% to 22.75%. In absolute values, the culture grown, before proceeding to the cells lysis, presents between 9E+10 and 9E+11 cells, preferably between 2E+11 and 8E+11, most preferably approximately 5E+11. In a particular embodiment, the species of *Saccharomyces* is *Saccharomyces cerevisiae.*

In particular embodiments of the present invention, one starts with a number of bacteria of the genus *Streptococcus* comprising between 2.72% to 28.15% of CFU with respect to the total CFU of the composition, preferably between 5.98% to 10.75%. In absolute values, the culture grown, before proceeding to the lysis of the cells, presents between 1E+11 and 6E+11 cells, preferably between 2E+11 and 4E+11, most preferably approximately 3.00E+11. In a particular embodiment, the species of *Streptococcus* is *Streptococcus thermophilus.*

These probiotic bacteria are cultured under standard conditions, as set out in the culture protocols published by the Spanish Collection of Type Cultures (CECT), indicated for each of the bacterial species described in this document.

Once these microorganisms are cultured, they are subjected to a lysis procedure. First of all, the microbial cells undergo heat treatment. Each batch of viable cell culture undergoes a sterilization cycle in an autoclave at 121°C for 20 to 30 minutes. This temperature denatures and coagulates the proteins by inactivating them. In addition, it causes damage to membranes, aggregation of ribosomes, breaking of DNA strands and inactivation of enzymes. Once cold, they undergo a cell rupture treatment by sonication for a period of 15 to 20min, at an output power of between 450 to 550 W, at an amplitude of between 38 to 43% and a period of 8 to 15 seconds of pause, obtaining a mass of lysed cells from the probiotic batch (Qsonica, Q500). This achieves the breakage of intermolecular interactions and DNA fragmentation. Optionally, the final solution is freeze-dried and ground to obtain a lysate of the powdery probiotic microorganism. The powder is kept in a cool environment away from heat.

The composition, in accordance with the invention, is the result of the combination of probiotic microorganisms that, after a process of growth and lysis, generates an extract consisting of a set of metabolites, proteins, DNA fragments and other components, such as, for example, peptidoglycans, which through efficient dosing is able to alter and modify in a surprising way the microbiota of the host through several mechanisms in such a way that it activates the immune system, reversing dysbiosis and surprisingly helping to improve a dyslipidemia.

The technical effect deriving from the composition of the invention is that it acts directly on the microbiota of a subject. Thanks to this restorative and modulating action, the inventors have confirmed that, surprisingly, intervening on the microbiota of a subject can significantly improve the health state of said subject, and in particular the condition of a dyslipidemia, as demonstrated by the experimental results described in the present application.

In a particular embodiment, when the composition of the invention is in powder form, the composition, in line with the first aspect of the invention, can be presented in sealed sachets, which is in itself conventional in the food and pharmaceutical industry.

In the context of the present invention, a dose is defined as the amount of the composition of the invention that is to be effective, efficacious and safe for the subject and to solve the health problem for which it has been indicated.

Thus, in the context of the present invention, it is established that at least one dose must be administered, preferably at least one daily dose, between 100mg to 80000mg of the composition of the invention, including any of the compositions A-H. In preferred embodiments, a dosage may comprise between 100mg to 50000mg of the composition of the invention, including any of the compositions A-H; in another preferred embodiment, a dosage may comprise between 100mg to 2000mg of the composition of the invention, including any of the compositions A-H; in another preferred embodiment, a dosage may comprise between 100mg to 700mg of the composition of the invention, including any of the compositions A-H. In particular embodiments of the present invention, a dosage may comprise approximately 120mg, 130mg, 140mg, 150mg, 160mg, 170mg, 180mg, 190mg, 200mg 210mg, 220mg, 230mg, 240mg, 250mg, 260mg, 270mg, 280mg, 290mg, 300mg 310mg, 320mg, 330mg, 340mg, 350mg, 360mg, 370mg, 380mg, 390mg, 400mg, 410mg, 420mg, 430mg, 440mg, 450mg, 460mg, 470mg, 480mg, 490mg, 500mg, 510mg, 520mg, 530mg, 540mg, 550mg, 560mg, 570mg, 580mg, 590mg, 600mg, 610mg, 620mg, 630mg, 640mg, 650mg, 660mg, 670mg, 680mg, or 690mg of the composition of the invention, including any of the compositions A-H.

In a particular embodiment, you can administer at least one dose of the composition of the invention, including any of the compositions A-H, daily, or every other day, or every 3 or 4 days, following any of the concentrations of the preceding paragraph.

In a particular embodiment the dosage is at least 100 mg/day, or at least 100 md/day, or between 80 and 150 mg/day, or between 80 and 200 mg/day.

In another additional embodiment, the administration of the postbiotic composition of the invention, including any of the compositions A-H, is daily for at least 2 weeks, preferably daily for at least 3 weeks, even more preferably daily for at least 4 weeks, even more preferably daily for at least 5 weeks, even more preferably daily for at least 6 weeks, even more preferably daily for at least 7 weeks, even more preferably daily for at least 8 weeks, or daily for at least 9 weeks, or daily for at least 10 weeks, or daily for at least 11 weeks, or daily for at least 12 weeks.

Preferably the composition of the invention, including any of the compositions A-H, is administered in an amount between 100mg to 700mg per dose and between 1 to 6 doses per day for at least 2 weeks.

In another additional embodiment, the treatment with the postbiotic composition is carried out before, simultaneously or after the treatment of the patient with any other dyslipidemia treatment. When the treatment with the composition of the invention manages to improve the general condition of the patient, any other dyslipidemia treatment can be performed if the doctors consider that the patient can withstand them.

An additional object of the invention relates to a pharmaceutical formulation comprising an effective pharmaceutical amount of the composition, in accordance with the first aspect of the invention, and at least one pharmaceutically acceptable excipient for use in the treatment of dyslipidemia, wherein the postbiotic composition of the invention can act as an hypocholesterolemic agent. This term as used herein, refers to an agent which provokes a reduction in the blood levels of cholesterol.

In the context of the present invention, the expression "pharmaceutical composition" refers to a formulation that has been adapted to deliver a predetermined dose of one or more useful therapeutic agents to a cell, a group of cells, an organ, or a tissue.

The expression "effective pharmaceutical quantity", as used herein, is understood as a nontoxic amount, capable of providing a therapeutic effect. The exact amount required will vary from one subject to another, depending on the species, age and general condition of the subject, the severity of the disease being treated, the particular compound used, its mode of administration, and the like. Therefore, it is not possible to specify an exact "effective quantity". However, an appropriate effective amount can be determined by a skilled person through routine experimentation. In the context of compounds and compositions for the prevention of infection, the pharmaceutically effective quantity can refer to the amount necessary to relieve the symptoms of a dyslipidemia suffered by the subject and improve their quality of life.

Also, in the context of the present invention, "pharmaceutically acceptable excipient" means a therapeutically inactive substance that is said to be used to incorporate the active ingredient, and that is acceptable to the patient from a pharmacological/toxicological point of view and to the pharmaceutical chemist who manufactures it from a physical/-chemical point of view, with respect to composition, formulation, stability, patient acceptance and bioavailability.

The pharmaceutical formulation of the invention can be suited for topical administration or oral administration. This is, the at least one pharmaceutical acceptable excipient is suitable for topical or oral administration.

A topical administration is that is applied directly over the skin or the mucosa.

Formulations for topical administration can include ointments, lotions, creams, gels, drops, suppositories, sprays, serums, oils liquids, and powders. Conventional pharmaceutical carriers, aqueous, powder or oily bases, thickeners and the like can be necessary or desirable.

In a particular embodiment, the formulation is administered as a cream. It may contain anti-inflammatories such as aloe vera, skin regenerators such as vitamin D, antioxidants such as vitamin C, moisturisers such as glycerine, collagen stimulators such as glycolic acid, and anti-ageing ingredients such as peptides, retinol and hyaluronic acid, drainers such as plant extracts like ivy, moisturizers such as grape seed oils and thermogenics to promote lipid metabolism, such as caffeine

For example, the excipients that can be used are: water, Glycerin, Butyrospermun Parkii Butter, Cetyl Palmitate, Dimethicone, Sodium, Polyacrylate, Phenoxyethanol, linalool, Ctitronellol, alpha-isomethyl Ionone, geraniol, mentol, parfum, Vitamin D, Aloe Vera, Vitamin E, Almond oil.

Formulations for oral administration can include powders or granules, suspensions or solutions in water or non-aqueous media, capsules, sachets, or tablets. Thickeners, flavorings, diluents, emulsifiers, dispersing aids or binders can be desirable.

Another additional object of the invention relates to a food supplement comprising the composition of the invention for use in the treatment of a patient with a dyslipidemia, including any of the compositions "A-H".

In the context of the present invention, what is understood by food supplement is a food product whose purpose it is to supplement the normal diet, and which consists of concentrated sources of nutrients or other substances, that has a nutritional or physiological effect, in simple or combined form, and is marketed in dosed form, ie capsules, tablets, pills and other similar means, sachets of powders, ampoules of liquid, dropper bottles, and other similar forms of liquids and powders to be taken in small unit quantities, as defined in Directive 2002/46/EC of the European Parliament.

In a particular embodiment, the food supplement, in accordance with the invention, is in capsule form, such as conventional gelatin capsules, inside which the composition in powder form is contained.

In particular embodiments where the composition is presented in sealed sachets, said composition can be administered to the patient dissolved or suspended in a liquid, preferably in an aqueous liquid, and more preferably, in beverages such as fruit juices, milk, water. In addition, it can also be mixed with foods such as yoghurt, liquid yoghurt, soups, purees, creams, or porridges. These foods must be at optimum temperature to be consumed, and never heated after having added the composition of the invention.

As it will be shown later, the composition of the invention is effective as a food supplement orally administered.

The postbiotic composition of the invention being composed of lysates of microorganisms of multiple probiotic species and/or the formulation comprising said composition, is intended to prevent and/or treat, this is, to control or at least mitigate a dyslipidemia in a subject. As will be show later in the examples section, the postbiotic composition of the invention is able to treat or prevent hypercholesterolemia in an in vivo model, therefore, the postbiotic composition of the invention can act as an hypocholesterolemic agent.

The term "dyslipidemia", as used herein, refers to an abnormal amount of lipids and lipid proteins in the blood, that is, an amount of lipids that is increased or decreased with respect to normal values. The lipids and lipid proteins may be:

Cholesterol, which in abnormal amounts may cause hypercholesterolemia, including familiar hypercholesterolemia due to a defect on chromosome 19 (19p13.1-13.3), and hypocholesterolemia.

Glycerides, such as triclycerides, which in abnormal amounts may cause hypertriglyceridemia and hypotriglyceridemia.

Lipoproteins, such as LDL and HDL, which in abnormal amounts may cause hyperlipoproteinemia and hypolipoproteinemia

In a particular embodiment the dyslipidemia is hypercholesterolemia. The term "hypercholesterolemia", as used herein, refers to any medical condition wherein blood cholesterol levels are elevated above the clinically recommended levels. For example, if cholesterol is measured using low density lipoproteins (LDLs), hypercholesterolemia may exist if the measured LDL levels are above, for example, approximately 70 mg/dl. Alternatively, if cholesterol is measured using free plasma cholesterol, hypercholesterolemia may exist if the measured free cholesterol levels are above, for example, approximately 200-220 mg/dl.

In another particular embodiment the dyslipidemia is hypertriglyceridemia. The term "hypertriglyceridemia", as used herein, refers to high blood levels of triglycerides. Based on fasting levels, mild and moderate hypertriglyceridemia is diagnosed with triglyceride levels of 150-999mg/dl, and severe and very severe hypertriglyceridemia is diagnosed with triglyceride levels superior to 1,000 mg/dl.

In another particular embodiment the dyslipidemia is hyperlipoproteinemia. The term "hyperlipoproteinemia", as used herein, refers to abnormally elevated levels of any lipoprotein in blood. Lipoproteins include chylomicrons, VLDL, LDL, and IDL. High levels of lipoproteins are associated with atherosclerosis. Hyperlipoproteinemia is divided in the following five types:

Type I: Fat-induced hyperlipidemia or idiopathic familial hyperlipidemia, which is a rare condition caused by deficient or abnormal lipase, characterised by a level of triglycerides of 1,000-10,000 mg/dl and more.

Type II: Familial hyperbetalipoproteinemia and essential familial hypercholesterolemia because of deficient cell surface receptors, characterised by elevated cholesterol and triglycerides, elevated low-density lipoproteins (LDL) and very low-density lipoproteins (VLDL).

Type III: Familial broad-beta disease xanthoma tuberosum caused by a deficient low-density lipoprotein receptor, characterised by elevated cholesterol and triglycerides; elevated intermediate-density lipoproteins.

Type IV: Endogenous hypertriglyceridemia and hyperbetalipoproteinemia with an idiopathic cause, characterised by triglycerides levels lower than 1,000 mg/dL, normal cholesterol, elevated VLDL.

Type V: Mixed hypertriglyceridemia from defective triglyceride clearance, characterised by triglycerides levels higher than 1,000 mg/dL, elevated cholesterol, normal LDL.

Since the various dyslipidemias are involved in insulin resistance, probably due to low blood levels of HDL, the postbiotic composition of the invention is also useful in the treatment of insulin resistance, metabolic syndrome, hypertension or atherosclerotic plaques, these disease are closely related dyslipidemias

As used herein, "subject" or "patient" includes mammals (e.g., a human, horse, pig, rabbit, dog, sheep, goat, non-human primate, cow, cat, guinea pig or rodent), a fish, a bird or a reptile or an amphibian. The subject can be an invertebrate, more specifically an arthropod (e.g., insects and crustaceans). The term does not denote a particular age or sex. Thus, adult and newborn subjects, as well as fetuses, whether male or female, are intended to be covered. A patient refers to a subject afflicted with a disease or disorder. The term "patient" includes human and veterinary subjects. In one aspect, the compounds described herein can be administered to a subject comprising a human or an animal including, but not limited to, a mouse, dog, cat, horse, bovine or ovine and the like, that is in need of alleviation or amelioration from a recognized medical condition.

Therefore, the main advantages deriving from the postbiotic composition of the invention are the following:

-    the physiological functions of the microbiota of humans and animals are supplemented and/or complemented;

-    the alterations that lead to dysbiosis and have an impact on oxidative and inflammatory processes are restored;

-    the fact that the product is administered topically, makes the present composition, and its surprising effect, a very advantageous alternative to current dyslipidemia treatments;

-    the present composition comprises lysates of microorganisms, i.e. it does not contain living organisms, which makes the present composition a safe alternative, because it avoids the possible dangers of colonising organisms, and it does not have the toxicity that a conventional drug can have.

Additionally, the invention comprises the following clauses:

1. A postbiotic composition that comprises lysates of probiotic microorganisms of the genus *Bacillus, Bifidobacterium,*

*Lactobacillus, Saccharomyces* and *Streptococcus* for use in the treatment and/or prevention of a dyslipidemia in a subject.

2. The postbiotic composition for use, according to the preceding clause, wherein the bacterial lysates of the genus *Bacillus* are of the species that are selected from the group consisting of *Bacillus clausii, Bacillus coagulans, Bacillus licheniformis, Bacillus pumilus, Bacillus subtilis, Bacillus mesentericus Bacillus paralicheniformes,* and combinations thereof, preferably *Bacillus licheniformis, Bacillus coagulans* and *Bacillus subtilis.*

3. The postbiotic composition for use, according to any of the preceding clauses, wherein the bacterial lysates of the genus *Bifidobacterium* are of the species *Bifidobacterium animalis subsp lactis, Bifidobacterium bifidum, Bifidobacterium breve, Bifidobacterium lactis, Bifidobacterium longum, Bifidobacterium infantis, Bifidobacterium animalis,* and combinations thereof, preferably *Bifidobacterium bifidum, Bifidobacterium breve, Bifidobacterium lactis and Bifidobacterium animalis subsp lactis.*

4. The postbiotic composition for use, according to any of the preceding clauses, wherein the bacterial lysates of the genus *Lactobacillus* are of the species that are selected from the group consisting of *Lactobacillus lactis, Lactobacillus acidophilus, Lactobacillus bulgaricus, Lactobacillus casei, Lactobacillus fermentum, Lactobacillus paracasei, Lactobacillus plantarum, Lactobacillus reuteri, Lactobacillus rhamnosus Lactobacillus salivarius, Lactobacillus helvéticus, Lactobacillus gasseri, Lactobacillus johnsonii, Lactobacillus brevis, Lactobacillus kefiri,* and combinations thereof, preferably *Lactobacillus acidophilus, Lactobacillus casei, Lactobacillus plantarum, Lactobacillus reuteri* and *Lactobacillus rhamnosus.*

5. The postbiotic composition for use, according to any of the preceding clauses, wherein the lysates of yeast genus *Saccharomyces* are of the species that are selected from the group consisting of *Saccharomyces cerevisiae, Saccharomyces boulardii,* and combinations thereof, preferably *Saccharomyces cerevisiae.*

6. The postbiotic composition for use, according to any of the preceding clauses, wherein the bacterial lysates of the genus *Streptococcus* are of the species that are selected from the group consisting of *Streptococcus thermophilus, Streptococcus salivarius,* and combinations thereof, preferably *Streptococcus thermophilus.*

7. The postbiotic composition for use, according to the preceding clauses 1 to 6 wherein the composition comprises lysates of probiotic microorganisms of *Bacillus licheniformis, Bacillus subtilis, Bacillus coagulans, Bifidobacterium bifidum, Bifidobacterium breve, Bifidobacterium lactis, Bifidobacterium animalis susp lactis, Lactobacillus acidophilus, Lactobacillus casei, Lactobacillus plantarum, Lactobacillus reuteri, Lactobacillus rhamnosus, Saccharomyces cerevisiae* and *Streptococcus thermophilus.*

8. The postbiotic composition for use, according to the preceding clause, wherein the percentage by weight of *Bacillus licheniformis* and *Bacillus subtilis* is approximately the same, and is higher than that of *Bacillus coagulans,* or, the percentage by weight of *Bacillus licheniformis, Bacillus coagulans* and *Bacillus subtilis* is approximately the same.

9. The postbiotic composition for use, according to any of the preceding clauses 7 or 8, wherein the percentage by weight of *Bifidobacterium animalis susp lactis* and *Bifidobacterium breve* is approximately the same, and is higher than that of *Bifidobacterium bifidum* and *Bifidobacterium lactis,* or, the percentage by weight of *Bifidobacterium animalis susp lactis* and *Bifidobacterium breve* is approximately the same, and is higher than that of *Bifidobacterium lactis* which is higher than that of *Bifidobacterium bifidum.*

10. The postbiotic composition for use, according to any of the preceding clauses 7 to 9, wherein the percentage by weight of *Lactobacillus rhamnosus* and *Lactobacillus acidophilus* is approximately the same, and is smaller than *Lactobacillus casei, Lactobacillus plantarum,* and *Lactobacillus reuteri,* or, the percentage by weight of *Lactobacillus rhamnosus* and *Lactobacillus acidophilus* is approximately the same, and is smaller than *Lactobacillus casei, Lactobacillus plantarum,* and *Lactobacillus reuteri* which is approximately the same.

11. The postbiotic composition for use, according to any of the preceding clauses, that comprises lysates of probiotic microorganisms in a percentage amount by weight with respect to the total weight of the lysates of microorganisms of the composition:

- 0.1% to 10% of bacterial lysates of the genus *Bacillus;*
- 8% to 40% of bacterial lysates of the genus *Bifidobacterium;*

- 8% to 60% of bacterial lysates of the genus *Lactobacillus;*
- 15% to 60% of yeast lysates of the genus *Saccharomyces;*
- 0.5% to 15% of bacterial lysates of the genus *Streptococcus.*

12. The postbiotic composition for use, according to any of the preceding clauses, that comprises lysates of probiotic microorganisms in a percentage amount by weight with respect to the total weight of the lysates of microorganisms of the composition:

- 0.1% to 10% of bacterial lysates of the genus *Bacillus;*
- 8% to 40% of bacterial lysates of the genus *Bifidobacterium;*
- 8% to 40% of bacterial lysates of the genus *Lactobacillus;*
- 20% to 60% of yeast lysates of the genus *Saccharomyces;*
- 0.5% to 15% of bacterial lysates of the genus *Streptococcus.*

13. The composition for use, according to any of the preceding clauses, that is administered orally or topically.

14. The composition for use, according to any of the preceding clauses, that comprises between 1% and 99.5% by weight of lysates of microorganisms, particularly 5%-99% by weight of lysates of microorganisms, particularly 10%-96% by weight of lysates of microorganisms, 25%-95% by weight of lysates of microorganisms, or particularly 50%+10% by weight of lysates of microorganisms.

15. The composition for use, according to any of the preceding clauses, wherein the bacterial lysates of the genus *Bacillus* are approximately 1% to 8% of the total lysates of the composition.

16. The composition for use, according to any of the preceding clauses, wherein bacterial lysates of the genus *Bifidobacterium* are approximately 12% to 35% of the total lysates of the composition.

17. The composition for use, according to any of the preceding clauses, wherein bacterial lysates of the genus *Lactobacillus* are about 12% to 35% of the total lysates of the composition.

18. The composition for use, according to any of the preceding clauses, wherein the lysates of yeast of the genus *Saccharomyces* are approximately between 25% to 50% of the total lysates of the composition.

19. The composition for use, according to any of the preceding clauses, wherein the lysates of yeast of the genus *Streptococcus* are approximately between 1% to 10% of the total lysates of the composition.

20. The composition for use, according to any of the preceding clauses, wherein the composition comprises lysates of probiotic microorganisms in an amount in percentage by weight of the total lysates of the composition according to composition A.

21. The composition for use, according to any of the preceding clauses, wherein the composition comprises lysates of probiotic microorganisms in an amount in percentage by weight of the total lysates of the composition according to composition B.

22. The composition for use, according to any of the preceding clauses, wherein the composition comprises lysates of probiotic microorganisms in an amount in percentage by weight of the total lysates of the composition according to composition C.

23. The composition for use, according to any of the preceding clauses, wherein the composition comprises lysates of probiotic microorganisms in an amount in percentage by weight of the total lysates of the composition according to composition D.

24. The composition for use, according to any of the preceding clauses, wherein the composition comprises lysates of probiotic microorganisms in an amount in percentage by weight of the total lysates of the composition according to composition E.

25. The composition for use, according to any of the preceding clauses, wherein the composition comprises lysates of probiotic microorganisms in an amount in percentage by weight of the total lysates of the composition according to composition F.

26. The composition for use, according to any of the preceding clauses, wherein the composition comprises lysates of probiotic microorganisms in an amount in percentage by weight of the total lysates of the composition according to composition G.

27. The composition for use, according to any of the preceding clauses, wherein the composition comprises lysates of probiotic microorganisms in an amount in percentage by weight of the total lysates of the composition according to composition H.

28. A procedure for obtaining a postbiotic composition for use in the treatment of a dyslipidemia, according to any of the clauses 1 to 27 comprising:

- Cultivating the selected microbial species

- Filter or centrifuge the cultures until an adequate biomass of the selected microorganisms is obtained

- Lysis through cycles of freezing and thawing, and sonication of the obtained the biomasses

29. The process, according to the preceding clause, comprising drying by atomization or lyophilization of the biomasses of bacterial lysates obtaining a product in the form of dry powder.

30. A pharmaceutical formulation comprising an effective pharmaceutical quantity of the postbiotic composition, according to any of the preceding clauses 1 to 27, and at least one pharmaceutically acceptable excipient for use in the treatment of a dyslipidemia.

31. The pharmaceutical formulation for use, according to the preceding clause, wherein the at least one pharmaceutical acceptable excipient is suitable for topical or oral administration.

32. The pharmaceutical formulation for use, according to any of the preceding clauses 30 to 31, wherein formulations for topical administration are ointments, lotions, creams, gels, drops, suppositories, sprays, serums, oils liquids, and powders.

33. The pharmaceutical formulation for use, according to any of the preceding clauses 30 to 32, wherein the formulation is administered as a cream and comprises anti-inflammatories, antioxidants, moisturisers, collagen stimulators, drainers, moisturizers, thermogenics and anti-ageing ingredients.

34. The pharmaceutical formulation for use, according to any of the preceding clauses 30 to 31, wherein formulations for oral administration are powders or granules, suspensions or solutions in water or non-aqueous media, capsules, sachets, or tablets

35. A food supplement comprising the postbiotic composition, according to any of the clauses 1 to 27, for use in the treatment of a dyslipidemia in a subject.

36. The composition for use, according to any of the preceding clauses 1 to 27, the pharmaceutical formulation for use, according to any of the preceding clauses 30 to 34 and/or the food supplement for use, according to clause 35, wherein the dyslipidemia is hypercholesterolemia.

Each of the terms "comprising," "consisting essentially of," and "consisting of" may be replaced with either of the other two terms. The term "a" or "an" can refer to one of or a plurality of the elements it modifies (e.g., "a reagent" can mean one or more reagents) unless it is contextually clear either one of the elements or more than one of the elements is described. The term "approximately" or "about" as used herein refers to a value within 10% of the underlying parameter (i.e., plus or minus 10%; e.g., a weight of "about 100 grams" can include a weight between 90 grams and 110 grams). Use of the term "about" at the beginning of a listing of values modifies each of the values (e.g., "about 1, 2 and 3" refers to "about 1, about 2 and about 3"). When a listing of values is described the listing includes all intermediate values and all fractional values thereof (e.g., the listing of values "80%, 85% or 90%" includes the intermediate value 86% and the fractional value 86.4%). When a listing of values is followed by the term "or more," the term "or more" applies to each of the values listed (e.g., the listing of "80%, 90%, 95%, or more" or "80%, 90%, 95% or more" or "80%, 90%, or 95% or more" refers to "80% or more, 90% or more, or 95% or more"). When a listing of values is described, the listing includes all ranges between any two of the values listed (e.g., the listing of "80%, 90% or 95%" includes ranges of "80% to 90%", "80% to 95%" and "90% to 95%"). Certain implementations of the technology are set forth in examples below

## BRIEF DESCRIPTION OF THE FIGURES

To help a better understanding of the invention, a set of figures is included as an integral part of this description, where, for illustrative and non-limiting purposes, graphs of the experimental results obtained in the following examples are presented in this document.

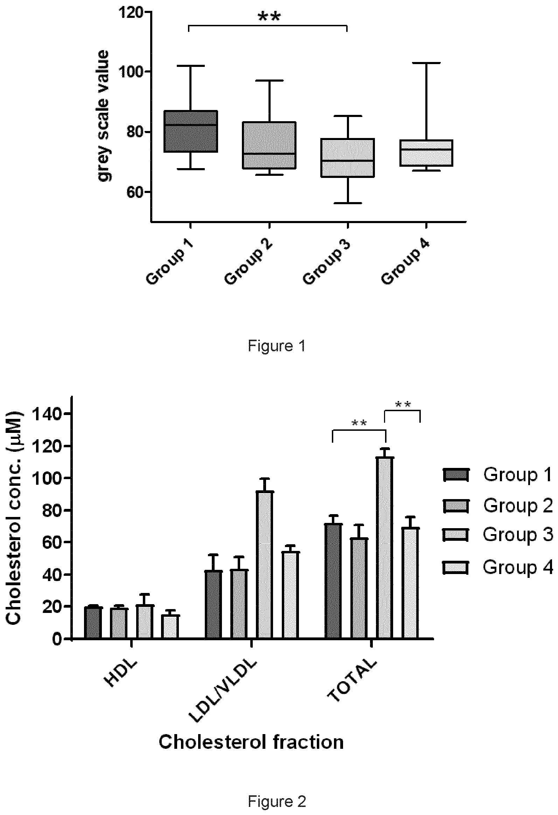

**Figure 1.** Box plot of the grey scale values of the four different test Groups 1-4 with the respective standard deviation. Each box represents the 25th - 75th percentiles with the line as the median, and the maximum and minimum values as whiskers. A statistically significant difference was detected between Group 1 and 3 indicated with "**" by a t-test ($p \leq 0.01$).

**Figure 2.** Cholesterol concentrations ($\mu$M) of the experimental Groups 1 - 4 measured as the sub-fractions of high-density (HDL), low-density and very low-density lipoprotein (LDL/VLDL) and the calculated sum amounts (total). The bars represent the mean $\pm$ standard deviation of three replicates of five pooled larvae. "**" t-test p values $\leq 0.01$. The order of the columns in HDL, LDL/VLDL and TOTAL is group 1, group 2, group 3 and group 4.

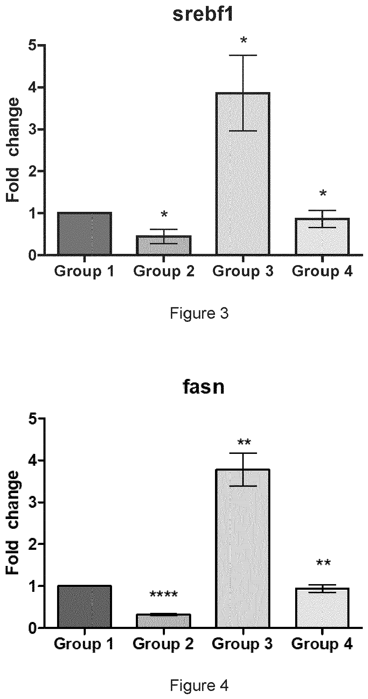

**Figure 3.** Expression levels of *srebf1 (sterol regulatory element binding transcription factor 1; Gene ID:* 793274). Values (Mean $\pm$ SEM) of fold change were compared between control Group 1 (for Groups 2 and 3) and Group 3 (for Group 4). Statistically significant difference between Groups 1 and 3; Groups 1 and 2; and Groups 3 and 4 by t-test; *: p < 0.05.

**Figure 4.** Expression levels of *fasn (fatty acid synthase; Gene ID: 559001)*. Values (Mean $\pm$ SEM) of fold change were compared between control Group 1 (for Groups 2 and 3) and Group 3 (for Group 4). Statistically significant difference between Groups 1 and 3; Groups 1 and 2; and Groups 3 and 4 by t-test; **: p < 0.01; ****: p < 0.0001.

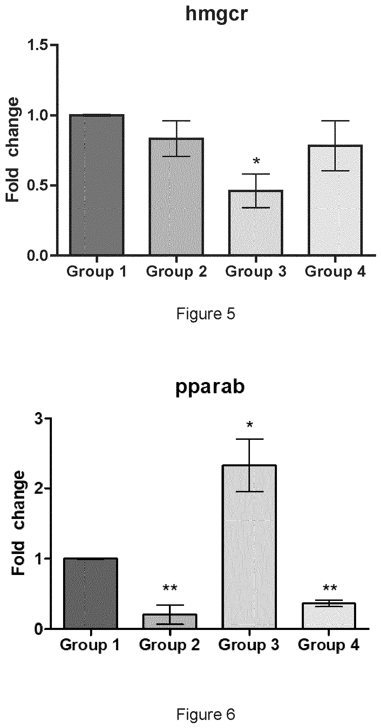

**Figure 5.** Expression levels of *hmgcr (3-hydroxy-3-methylglutaryl-CoA reductase; Gene ID:* 559054). Values (Mean $\pm$ SEM) of fold change were compared between control Group 1 (for Groups 2 and 3) and Group 3 (for Group 4). Statistically significant difference between Groups 1 and 3 by t-test; *: p < 0.05.

**Figure 6.** Expression levels of *pparab (peroxisome proliferator-activated receptor alpha b; Gene ID: 557714)*. Values (Mean $\pm$ SEM) of fold change were compared between control Group 1 (for Groups 2 and 3) and Group 3 (for Group 4). Statistically significant difference between Groups 1 and 3; Groups 1 and 2; and Groups 3 and 4 by t-test; **: p < 0.01; *: p < 0.05.

## EXAMPLES

So as to contribute to a better understanding of the invention, and in accordance with a practical embodiment thereof, a preferred embodiment of the present invention is attached hereto as an integral part of this description.

## EXAMPLE 1: EFFECT OF THE POSTBIOTIC COMPOSITION OF THE INVENTION IN A HYPERCHOLESTEROLEMIC MODEL OF ZEBRAFISH

### Objective

This study aimed to evaluate the effect of the postbiotic composition of the invention in Zebrafish juvenile by an hypercholesterolemic model by using a high cholesterol diet (HCD) on preventing hypercholesterolemia via inspection of the lipid accumulation by a specific staining, HDL - LDL/VLDL measurements, and expression of gene involved in lipid metabolism. The postbiotic composition of the invention was provided in the diet.

### Material and methods

*Postbiotic composition of the invention:*

A postbiotic composed of lysates from 14 strains/species of *Lactobacillus sp., Bifidobacterium sp., Bacillus sp,*

*Saccharomyces sp.* and *Streptococcus sp.* was used. The composition of the extracts appears in Table 2 in an amount in percentage by weight over the total weight of the lysates of the composition and CFU percentage over the total CFUs.

Table 2: Components of the postbiotic composition of the invention

| Species/Strains | % (w/w) | % CFU |
|---|---|---|
| *Bacillus coagulans* | 0,50 | 0,95 |
| *Bacillus licheniformis* | 0,67 | 1,27 |
| *Bacillus subtilis* | 0,67 | 1,27 |
| *Bifidobacterium animalis susp lactis* | 8,39 | 3,16 |
| *Bifidobacterium bifidum* | 1,68 | 3,16 |
| *Bifidobacterium breve* | 8,39 | 3,16 |
| *Bifidobacterium lactis* | 4,19 | 7,91 |
| *Lactobacillus acidophilus* | 1,68 | 3,16 |
| *Lactobacillus casei* | 8,39 | 15,82 |
| *Lactobacillus plantarum* | 8,39 | 15,82 |
| *Lactobacillus reuteri* | 1,68 | 3,16 |
| *Lactobacillus rhamnosus* | 8,39 | 15,82 |
| *Saccharomyces cereviseae* | 41,95 | 15,82 |
| *Streptococcus thermophilus* | 5,03 | 9,49 |

Unless otherwise specified, the composition of Table 2 was used in all experiments. The lyophilized powder of the composition of the invention was kept at 4°C until used.

In the present specification, the expressions "postbiotic composition of the invention", "active ingredient" and "test item" are synonymous and are used interchangeably

*Animal care and embryo obtaining*

Adult zebrafish were housed and maintained following standard procedures. Briefly, fish were maintained in 3-liter aquaria heated to 28.5 ∘C, with about 20 fish per tank and water continuously filtered. Water was maintained at pH 7-7.8, conductivity at 500-800 $\mu$S, and O2 saturation between 80-100%. Water conditions were monitored and regulated conveniently. Fish were kept under a photoperiod of 14:10 hours light:dark. Adults were fed with ground dry pellets and live food following standard procedures.

Embryos were collected following standard procedures, raised in E3 medium with ampicillin (100 $\mu$g/ml) and methylene blue (0.0001%), and kept in plates in the incubator at 28.5 °C until they reached 5 dpf (days post fertilization). At this stage, the larvae require an external supply of nutrients as the independent feeding from the yolk sac is over.

*Materials Equipment and Reagents*

*Materials*

- Plastic tips
- 1.5 mL microtubes
- 15 and 50 mL Falcon tubes
- Pasteur pipets
- Petri dishes
- Fish tanks and nests
- Pellet pestle sticks
- Protective equipment, PNT-BBD-PRL-011.
- 0.2 mL PCR tube strips

- Microscope slides
- qPCR 96-well plates and seals
- Glass vials

*Equipment*

- Vortexes
- Autoclave
- Scales:
- Incubators at 28.5 °C
- -20 °C freezers
- -80 °C freezers
- 5 °C refrigerators
- Microcentrifuges
- Refrigerated microcentrifuge
- Micropipette sets
- Dispensing pipette:
- Fume hoods
- Milli-Q water/ ELIX water system
- Ice machine
- Sonicator
- Fish tank system
- Cytation 3 plate reader
- AriaMx Real Time PCR System
- Thermomix Compact
- Biodrop
- Stereoscope
- Agilent Aria 1.5 AriaMx Real-Time PCR Software
- Amscope (ToupTek XCamView, V2.4_20170904)
- BioTek Gen5
- GraphPad (Version 9)
- ImageJ (1.53q)

*Reagents*

Reagents used are shown in Table 3

Table 3: List of reagents used

| Product | Brand / Cat N / Batch | Storage conditions | Vehicle |
|---|---|---|---|
| Ampicillin sodium salt | Sigma-Aldrich / A9518 / BCCC0880 | 5 ± 3°C in solid state / -20°C in solution. | MilliQ water |
| E3 Medium | N/A | RT, tightly closed. | N/A |
| Magnesium sulphate heptahydrate | Sigma-Aldrich / M5921/ L1003338869 | RT, tightly closed. | MilliQ water |
| Calcium chloride | Sigma-Aldrich / 223506 / BCCF9439 | RT, tightly closed. | MilliQ water |
| Potassium chloride | Sigma-Aldrich / 746436 / SZBF2720V | RT, tightly closed. | MilliQ water |

(continued)

| Product | Brand / Cat N / Batch | Storage conditions | Vehicle |
|---|---|---|---|
| Sodium chloride | Sigma-Aldrich / S6191 / SLCC5279 | RT, tightly closed. | MilliQ water |
| Methylene blue | Acros Organics / 229801000 / A0331880 | RT, tightly closed. | MilliQ water |
| Hepes | Sigma-Aldrich / H3375 / SLCH5583 | RT, tightly closed. | MilliQ water |
| DMSO | Scharlab / SU01531000 / 21743805 | RT, protected from direct exposure to natural light. | N/A |
| Tricaine | Sigma-Aldrich / E10521 / WXBC5194V | RT | MilliQ water |
| NaOH | Scharlab / SO04250500 / 20890902 | RT, tightly closed, protected from direct exposure to natural light. Do not store with acids. Do not store in aluminum containers, tin or zinc. | MilliQ water |
| Phosphate-buffer saline (PBS) | Fisher / BP399-4 / 219012 | RT, tightly closed. | N/A |
| Diethyl ether | Sigma-Aldrich / 179272 / STBK2796 | RT, tightly closed. | Diethyl ether |
| Butylhyrodxytoluene (BHT) | Sigma-Aldrich / B1215000 | RT, tightly closed | Ethanol |
| Ethanol | Scharlab / ET0002 / 18765803 | RT, tightly closed | N/A |
| Vegetable oil | KOIPE SOL / AO1967 / L24173 | RT | N/A |
| HDL and LDL/VLDL Cholersterol Assay Kit | Cell Biolabs, INC. / STA-391 / 92820221 | 4°C | N/A |
| Superscript IV | Invitrogen/ 01261666/ 18090010 | -20°C | N/A |
| RNAqueous kit | Invitrogen / AM1912 / 01269881 | 4°C | N/A |
| Brilliant III Ultra-Fast SYBR Green | Agilent/ 600882/ 0006649797 | -20°C | N/A |
| KiCqStart Primers | Sigma (KSPQ12012G) | -20°C | Sterile water |
| Paraformaldehyde (4%) in PBS | Fisher / 15670799/ 206909 | 4°C | PBS |

(continued)

| Product | Brand / Cat N / Batch | Storage conditions | Vehicle |
|---|---|---|---|
| Oil Red O | Sigma-Aldrich / O1516/ SLCF8267 | RT | PPG (see deviation 1) |
| Glycerol | Sigma-Aldrich / G5516 / SHBG8251V | RT | N/A |
| Polypropylene glycol | Sigma Aldrich / P4347-500ML/ MKBZ1609V | RT | N/A |
| Methylcellulose | Sigma Aldrich / M0512 / SLCD2238 | RT, tightly closed. | MilliQ water |
| [1] E3 content (0.16 mM $MgSO_4$, 0.4 mM $CaCl_2$, 0.17 mM KCl, 5 mM NaCl). E3 medium to be used in this Study is buffered to pH 7.2-7.6 with 10 mM HEPES. | | | |

## LIPID STAINING ASSAY, HDL and LDUVLDL, AND GENE EXPRESSION ANALYSIS

## TESTING CONDITIONS

- Sample size: 90 wild type zebra fish per experimental group were distributed in 2 tanks (1 tank with 2 nests + 1 tank with 1 nest with 30 larvae/nest). A total number of 8 tanks were deployed for the 360 wild type larvae.

- Four experimental groups, each one corresponding to two 3-liter tanks as follows:

    ◦ Group 1: Normal diet + no active ingredient

    ◦ Group 2: Normal diet + postbiotic composition

    ◦ Group 3: HCD + no active ingredient

    ◦ Group 4: HCD + postbiotic composition

- Feeding with the test diets took place from 5 dpf to 14 dpf, 3 times per day.

- All tanks have received a manual water replacement of 10% volume every day until 14 dpf and were incubated at 28.5 °C with a light-dark cycle of 14:10 hours.

- At 15 dpf larvae were euthanized and stained with Oil Red O to evaluate lipid accumulation via microscopy. The staining intensities of the different treatments were compared

- At 15 dpf larvae were euthanized and HDL and LDL/VLDL was quantified from whole larvae homogenates using a commercially available kit.

- At 15 dpf larvae were euthanized and the relative expression levels of the target genes *srebf1, fasn, hmgcr, pparab* and *ef1-alpha* were assessed by RT-qPCR.

## PROTOCOL

### Diet preparation

To prepare the 4 % supplemented cholesterol-enriched diet (HCD), a 10% wt/v cholesterol solution was generated in diethyl ether. 1840 mg of the standard zebrafish larva food (Sera micron) was then mixed with 1600 µL of the 10% wt/v cholesterol solution. After well mixing, the HCD was left at room temperature overnight for the ether to evaporate. After drying, the test diet HCD + postbiotic composition was prepared by mixing 500 mg HCD with 500 mg of the lyophilized postbiotic composition (diet for Group 4). In contrast, 500 mg HCD was mixed with 500 mg of the standard zebrafish larva food (Sera micron) to achieve the HCD + no active ingredient (diet of Group 3).

The diets without cholesterol were prepared by mixing 1840 mg of larva food with 1600 µL of pure diethyl ether. After overnight drying, 500 mg were mixed with 500 mg of the postbiotic composition to achieve the normal diet + postbiotic composition (diet for Group 2). For the normal diet of Group 1, no active ingredient was added, and 500 mg of the dry diet was mixed with 500 mg of the standard zebrafish larva food (Sera micron). Finally, all diets of Group 1-4 were spray-coated with vegetable oil and left to dry overnight at room temperature in a dry and dark place. The next day, the test diets were aliquoted and kept at 4 °C for the duration of the trial to avoid the degradation of the cholesterol and the test item.

### Zebrafish housing and feeding regime

Wild-type embryos were obtained following standard protocols in the field. Synchronized embryos were supplied to the laboratory and kept at 28.5 °C in an incubator until they reached 5 dpf. At that stage, larvae were transferred to 3-liter aquarium, distributed in the different nests as indicated above and were kept under a photoperiod of 14:10 hours light dark cycle. Tanks were housed in an independent incubator. The zebrafish larvae were fed with the corresponding diets prepared as described previously from 5 - 14 dpf. The larvae in the individual nests were fed three times a day with 6 mg/nest.

Fish welfare was checked at each feeding time and at the end of the workday, and dead larvae were removed daily.

### Sample collection

At the end of the experimental trial, 15 dpf wild type larvae were euthanized in a solution containing 0.25 mg/mL Tricaine in E3 medium and samples were collected for various analyses. For each experimental group, pools of 5 larvae taken from the identical nest were collected, cooled down in ice water and flash-frozen in liquid nitrogen for gene expression analysis and for quantification of HDL - LDL/VLDL (at least three pools for each assay). Finally, 15 larvae were used for whole-mount lipid staining for group 2 and group 4 while 14 and 13 larvae were used for group 1 and 3, respectively. The remaining larvae that served as back-ups in case of mortality were eliminated.

### Whole-mount lipid staining (PNT-BBD-LAB-072)

For the whole-mount lipid staining, the larvae were fixed in 4% wt/v paraformaldehyde (PFA). The larvae were then dehydrated in a series of propylene glycol (PPG) - phosphate buffer saline (PBS) solution of increasing PPG concentration. Finally, samples in 100% v/v PPG were stained applying a 0.5% wt/v Oil Red O solution. After staining, the samples were brought back to the aqueous phase. The stained samples were placed on a microscope slide embedded with 3% wt/v Methylcellulose, and the larvae were imaged with a Zeiss stereomicroscope using brightfield illumination.

### Quantification of HDL and LDL/VLDL

The collected pools of 5 larvae were homogenized in PBS containing BHT (10 µg/mL) using a pestle. Homogenates were centrifuged at 4 °C at 2000 g for 5 minutes to pellet the debris. The supernatant was mixed with an equal volume of precipitation solution from the commercial kit (Quimigen, STA-391) to separate the high density lipoprotein (HDL) and low density lipoprotein (LDL) fractions. Each lipoprotein fraction was mixed with an equal volume of reaction mix and quantified in a Cytation 3 plate reader (excitation 530-570 nm, emission 590-600 nm).

### Gene expression analysis

The RNA from the frozen pooled larvae samples was extracted using the RNAqueous kit (Ambion) following the manufacturer instructions. RNA samples were retrotranscribed into cDNA using the Superscript IV (Invitrogen), following the manufacturer instructions.

Gene-specific primers for *srebf1, fasn, hmgcr, pparab* and *ef1-α* (housekeeping gene) were used. The respective primer sequences are disclosed in Table 4

Table 4: List of primers used

| Name | PRIMER SEQUENCE (5'→3') | |
|---|---|---|
| ZF-*ef1-α* | Forward | GAACGACCCACCCATGGAGG |
| | Reverse | TGATGACCTGAGCGTTGAAG |
| ZF_*srebf1* | Forward | CATCCACATGGCTCTGAGTG |
| | Reverse | CTCATCCACAAAGAAGCGGT |
| ZF_*fasn* | Forward | GAGAAAGCTTGCCAAACAGG |
| | Reverse | GAGGGTCTTGCAGGAGACAG |
| ZF_*hmgcr* | Forward | CTGAGGCTCTGGTGGACGTG |
| | Reverse | GCAGCTACGATGTTGGCG |
| ZF_*pparab* | Forward | CGTCGTCAGGTGTTTACGGT |
| | Reverse | AGGCACTTCTGGAATCGACA |

Forward and reverse primers were combined with SYBR green and cDNA. The mixture was analysed in triplicates by quantitative real-time PCR in an AriaMx Real-Time PCR System to assess the expression levels of srebf1, fasn, hmgcr, pparab and ef1- α (the latter as a housekeeping gene). At the end of the PCR program, a melting curve analysis was performed in order to assess the specificity of the primers.

VALIDITY CRITERIA, DATA ANALYSIS, AND INTERPRETATION OF RESULTS

VALIDITY CRITERIA

The survival of the larvae was evaluated daily from 5 to 15 dpf. The experiment was evaluated valid, since the percentage of mortality in any of the experimental groups did not exceed 50 %.

DATA ANALYSIS

Image Analysis of Lipid Staining

The images of the whole-mount lipid staining were evaluated for differences in staining intensities and/or areas based on imaged analysis.

Quantification of HDL and LDL/VDL

Analysis of HDL and LDL/VLDL cholesterol was performed in a Cytation 3 plate reader. A cholesterol standard curve was prepared together with the samples. The concentration of cholesterol within the samples was calculated based on the cholesterol standard curve using the sample RFU (Relative Fluorescent Unit).
Samples and standard curves were run in duplicates and the averaged fluorescent values were corrected by subtracting the average zero standard value.
Values obtained from the standard curve were plotted to calculate a linear regression to obtain the slope.
Cholesterol contained in each sample was calculated using the following equation:

$$Total, HDL \text{ or } LDL/VLDL \text{ } (\mu M) = (sample \text{ } RFU/slope) \text{ } x \text{ } sample \text{ } dilution$$

Bar plots were generated to indicate the concentrations of HDL and LDL/VLDL of each experimental group.

Gene expression analysis

Analysis of the gene expression data was performed in the AriaMx Real-Time PCR Software. Relative expression levels of srebf1, fasn, hmgcr, and pparab were assessed using the $2^{-\Delta\Delta Ct}$ method.

First, the expression of these genes was normalized to the housekeeping gene ef1$\alpha$

Second, the relative expression level of each sample was calculated as a fold-change value relative to the control group. The control group was Group 1 (normal diet + no active ingredient).

The gene expression results are indicated as relative expression plots for all analysed genes and groups.

Results and conclusions

SECTION 1: LIPID STAINING ASSAY, HDL and LDUVLDL, AND GENE EXPRESSION ANALYSIS

**Whole-mount lipid staining assay**

For the whole-mount lipid staining assay, the sampled larvae were fixed and stained for lipid content using Oil Red O (ORO). Then images were taken at the Zeiss stereomicroscope and analysed using imaged to determine the mean grey scale value of the liver region. The resulting mean grey scale values of the lipid staining assay have to be considered as inverse staining intensities since lower grey values indicate high contrast liver images (i.e. higher contrast due to staining accumulation) and were presented in Table 5.

Table 5. Quantification of total lipid content

| Grey scale values of lipid staining assay | Group 1 (normal diet + no active ingredient) | Group 2 (normal diet + active ingredient) | Group 3 (HCD + no active ingredient) | Group 4 (HCD + active ingredient) |
|---|---|---|---|---|
| mean | 81.6 | 75.8 | 70.7 | 76.1 |
| SD | 9.6 | 9.2 | 8.7 | 9.7 |
| CV (%) | 11.8 | 12.1 | 12.4 | 12.8 |
| SEM | 2.6 | 2.4 | 2.4 | 2.5 |
| n | 14 | 15 | 13 | 15 |
| Mean grey scale values (possible range between 0-255, 0 = black=fully contrasted by ORO staining, 255= white = no contrast due to absence of ORO staining) with the respective standard deviation (SD), coefficient of variation (CV) in percent (%), standard error of the mean (SEM), and "n" as number of larvae. | | | | |

Zebrafish larvae that were fed a diet containing the postbiotic composition (Group 2) did not show any statistically significant difference in the lipid content in the liver measured as staining intensities of ORO compared to the normal diet-fed Group 1 (t-test, p > 0.05) (Table 5 and Figure 1). This indicated that feeding the postbiotic composition between 5-14 dpf did not negatively affect the lipid metabolism in zebrafish larvae (p > 0.05). Feeding a high cholesterol diet (HCD) caused an accumulation of ORO staining in the larvae livers of Group 3 compared to Group 1 which was indicated by lower mean grey scale values (Table 5). The accumulated ORO staining of Group 3 could be attributed to the HCD treatment and indicated a successful induction of the hypercholesterolemia disease model since the lipid increase was statistically significant different to the normal diet-treated Group 1 with p < 0.01 applying a t-test (Figure 1). The lipid accumulation of Group 3 was more prominent than in Group 4, however a statistically significant difference between these two groups could not be detected (Table 5).

Quantification of **HDL** and **LDL/VLDL**

To determine the cholesterol content of zebrafish larvae, the pooled larvae were homogenized and the high-density lipid (HDL), low-density lipid (LDL) and very low-density lipoprotein (VLDL) fractions were determined using a commercial kit. The total cholesterol concentrations of the larvae pools were calculated as the sum of the cholesterol sub-fractions and the mean values of the total cholesterol concentrations were compared between groups (Table 6 and Figure 2).

Table 6. Quantification of HDL and LDL/VLDL

| Cholesterol concentrations (µM) | Cholesterol fraction | Biological Replicates | | | mean | SD | SEM |
|---|---|---|---|---|---|---|---|
| | | 1 | 2 | 3 | | | |
| Group 1 (Normal diet + no active ingredient) | HDL | 105.0* | 19.3 | 20.7 | 20.0 | 0.9 | 0.7 |
| | LDL/VLDL | 24.8 | 57.5 | 46.5 | 42.9 | 16.6 | 9.6 |
| | Total | 129.7* | 76.8 | 67.2 | 72.0 | 6.8 | 4.8 |
| Group 2 (Normal diet + active ingredient) | HDL | 21.4 | 18.6 | 18.1 | 19.4 | 1.8 | 1.0 |
| | LDL/VLDL | 49.5 | 29.5 | 52.5 | 43.8 | 12.5 | 7.2 |
| | Total | 71.0 | 48.1 | 70.5 | 63.2 | 13.1 | 7.5 |
| Group 3 (HCD + no active ingredient) | HDL | 20.1 | 11.3 | 32.7 | 21.4 | 10.8 | 6.2 |
| | LDL/VLDL | 84.4 | 107.7 | 84.7 | 92.3 | 13.4 | 7.7 |
| | Total | 104.5 | 119.0 | 117.4 | 113.6 | 8.0 | 4.6 |
| Group 4 (HCD + active ingredient) | HDL | 16.7 | 18.5 | 9.8 | 15.0 | 4.6 | 2.6 |
| | LDL/VLDL | 54.8 | 60.7 | 48.7 | 54.7 | 6.0 | 3.5 |
| | Total | 71.5 | 79.2 | 58.5 | 69.8 | 10.4 | 6.0 |

Cholesterol concentrations (µM) measured in three biological replicates of pools of five larvae each. The high-density (HDL), low-density/very low-density lipoprotein (LDL/VLDL) and the calculated sum (total) fractions with the respective standard deviation (SD) and standard error of the mean (SEM) are presented for the different groups. "*" excluded outlier

Feeding the test active ingredient or postbiotic composition of the invention (Group 2) did not significantly alter the normal total cholesterol concentrations in 15 dpf old zebrafish larvae (Figure 2). Therefore, it was concluded that the postbiotic composition of the invention did not negatively affect normal lipid metabolism. A hypercholesterolemia disease model was induced by the administration of a HCD, which was indicated by a statistically significant difference between the Groups 1 and 3 for the total cholesterol fraction (t-test $p \leq 0.01$, Figure 2). The postbiotic composition of the invention had the potential to revert the hypercholesterolemia caused by HCD which could be detected by lower total cholesterol concentrations for Group 4 compared to Group 3. This difference was considered statistically significant since $p \leq 0.01$ (Figure 2). Noteworthy, the increase of the total cholesterol content (Group 3) and its reversal to control-like concentrations (Group 4) was attributed to the LDL/VLDL fraction only, which is generally considered to be the "bad" fraction of cholesterol building up plaques in blood vessels and inducing atherosclerosis.

**Gene expression analysis**

Total RNA was extracted with RNAqueous kit and three biological replicates were obtained for the four experimental groups and the mRNA was retrotranscribed to cDNA. Afterwards, cDNA was loaded in triplicates in 96-well PCR plates and run in a real-time AriaMX Real-Time PCR system using SYBR green to assess the expression levels of *srebf, fasn, hmgcr,* and *pparab* and *ef1a* as a housekeeping gene.
Melting curve analysis that was run after the amplification cycles were completed, confirmed the specificity of the primers for the evaluated genes, since only one clear peak was observed for all the samples in each case.
Relative expression levels for each gene were analysed with the $2^{-\Delta\Delta Ct}$ method, using the expression levels of *ef1a* as the housekeeping gene. First, the expression stability of *ef1a* was evaluated throughout all experimental diet groups, obtaining similar Cycle threshold (Ct) values for all of them.
Relative expression levels of target genes were analysed by the $2^{-\Delta\Delta Ct}$ method. The relative expression level of each sample was calculated as a fold change value relative to the control Group 1 (Table 7).

Table 7: Relative expression levels of target genes

| | Total Mean ± SEM | | |
|---|---|---|---|
| | Group 2 | Group 3 | Group 4 |
| srebf | 0.444 ± 0.172 | 3.860 ± 0.901 | 0.858 ± 0.205 |

(continued)

| | Total Mean ± SEM | | |
| --- | --- | --- | --- |
| | Group 2 | Group 3 | Group 4 |
| fasn | 0.316 ± 0.0225 | 2.682 ± 1.119 | 0.943 ± 0.091 |
| hmgcr | 0.836 ± 0.126 | 0.462 ± 0.122 | 0.781 ± 0.179 |
| pparab | 0.202 ± 0.133 | 1.576 ± 0.926 | 0.360 ± 0.046 |
| Expression levels of target genes in three experimental conditions. Ct results obtained from AriaMx Real-Time PCR system were used to calculate the mean fold change value ± SEM for three biological replicates relative to control Group 1 (normal diet + no active ingredient). | | | |

*srebf1 (sterol regulatory element binding transcription factor 1)*

Significant differences were detected between Groups 1 and 2 ($p < 0.05$). High relative expression levels of the transcription factor were obtained for Group 3 comparing with Group 1, showing a statistically significant difference ($p < 0.05$). When comparing HCD Groups 3 and 4, srebf1 levels were statistically significant downregulated in diet Group 4 ($p < 0.05$) (Figure 3). These results demonstrate that diets supplemented with the postbiotic composition of the invention reduced mRNA levels of genes involved in lipid synthesis.

*fasn (fatty acid synthase)*

High relative expression levels of the fasn enzyme were obtained for Group 3 comparing with Group 1, showing a statistically significant difference ($p < 0.01$) indicating an increased induction of the fatty acid synthase enzyme which is relevant for lipid biosynthesis. The increased expression levels in Group 3 support the induction of the hypercholesterolemia model. Expression levels of *fasn* showed a significant decrease in all probiotic supplemented diet when comparing with their respective control Groups (between Groups 1 and 2, $p < 0.0001$; between Groups 3 and 4, $p < 0.05$) (Figure 4). Therefore, as with the case of *srebf1,* diets supplemented with the postbiotic composition of the invention reduced mRNA levels of genes involved in lipid synthesis.

*hmgcr (3-hydroxy-3-methylglutaryl-CoA reductase)*

Statistically significant decreased levels of *hmgcr* were observed for HCD diet Group 3 when comparing with control Group 1 ($p < 0.05$) (Figure 5). Although no significant differences were detected in the relative gene expression levels for diet Groups 2 and 4 ($p > 0.05$), when comparing with their respective control Groups, a tendency to restore the expression levels of *hmgcr* in group 4 compared to group 3 is observed.

*pparab (peroxisome proliferator-activated receptor alpha b)*

More than 2-fold increase was detected for *pparab* relative expression for HCD diet Group 3 when comparing with control Group 1, showing a statistically significant difference ($p < 0.05$) supporting the successful induction of the hypercholesterolemia model. Expression levels of *pparab* showed a significant decrease in all probiotic supplemented diet when comparing with their respective control Groups (t-test: between Groups 1 and 2, $p < 0.01$; between Groups 3 and 4, $p < 0.01$) (Figure 6). Therefore, the postbiotic composition of the invention can be associated with a reduction in the lipid oxidation metabolism.

**Conclusions**

The present invention has unveiled the therapeutic use of a postbiotic composition in a hypercholesterolemia disease model of zebrafish larvae. This model was generated by feeding a high cholesterol diet and to evaluate the efficacy of the postbiotic composition of the invention to revert it. The observed endpoints where the whole-mount lipid staining assay, cholesterol measurements and gene expression analyses.

For the whole-mount lipid staining, the postbiotic composition of the invention fed to zebrafish larvae over 10 days (5-14 dpf) did not affect the normal liver lipid content. Staining intensities increased statistically significant when comparing the normal diet-fed Group 1 with the hyper cholesterol Group 3, which indicated a successful disease model induction. Although statistically non-significant, a slight reduction in the lipid content could be observed for the Group 4 which indicates a potential beneficial effect of the test item to reduce excessive lipid accumulation in zebrafish liver.

Similar to the staining assay results, no negative effect of the supplemented diet on the total cholesterol concentrations in zebrafish larvae could be revealed when comparing normal diet Group 1 and test item-fed Group 2. Also, the successful disease model induction could be confirmed by a statistically significant difference between the normal diet Group 1and the HCD-fed Group 3. The observed high cholesterol concentrations of the LDL/VLDL fraction of Group 3 were reverted by the administration of the test item via the diet in Group 4. This significant reduction in the cholesterol concentrations support a positive efficacy of the postbiotic composition of the invention.

In addition, samples from all Groups were obtained and expression levels of different inflammatory markers as well as genes involved in lipid metabolism were analysed after total RNA extraction and cDNA synthesis. Regarding the genes analysed after the feeding period, the test item-supplemented diet Groups 2 and 4 possessed lower mRNA levels of *pparab* (involved in lipid oxidation) and genes involved in lipid synthesis *(fasn* and *srebf1)* than the respective control Groups 1 and 3. The results demonstrate that the diet supplemented with the postbiotic composition of the invention and high cholesterol diet reduced both the hepatic catabolic and anabolic pathways.

Conclusively, the postbiotic composition of the invention showed a positive efficacy to prevent hypercholesterolemia in zebrafish larvae for the observed endpoints of total cholesterol concentrations and expression of genes involved in lipid metabolism.

## LIST OF SEQUENCES

(ZF-*ef1*-$\alpha$ Forward) SEQ_ID No 1: GAACGACCCACCCATGGAGG
(ZF-*ef1*-$\alpha$ Reverse) SEQ_ID No 2: TGATGACCTGAGCGTTGAAG
(ZF_ *srebf1* Forward) SEQ_ID No 3: CATCCACATGGCTCTGAGTG
(ZF_ *srebf1* Reverse) SEQ_ID No 4: CTCATCCACAAAGAAGCGGT
(ZF_*fasn* Forward) SEQ_ID No 5: GAGAAAGCTTGCCAAACAGG
(ZF_fasn Reverse) SEQ_ID No 6: GAGGGTCTTGCAGGAGACAG
(ZF_ *hmgcr* Forward) SEQ_ID No 7: CTGAGGCTCTGGTGGACGTG
(ZF_ *hmgcr* Reverse) SEQ_ID No 8: GCAGCTACGATGTTGGCG
(ZF_ *pparab* Forward) SEQ_ID No 9: CGTCGTCAGGTGTTTACGGT
(ZF_ *pparab* Reverse) SEQ_ID No 10: AGGCACTTCTGGAATCGACA

## Claims

1. A postbiotic composition that comprises lysates of probiotic microorganisms of the genus *Bacillus, Bifidobacterium, Lactobacillus, Saccharomyces* and *Streptococcus* for use in the treatment and/or prevention of a dyslipidemia in a subject.

2. The postbiotic composition for use, according to the preceding claim, wherein the bacterial lysates of the genus *Bacillus* are of the species that are selected from the group consisting of *Bacillus clausii, Bacillus coagulans, Bacillus licheniformis, Bacillus pumilus, Bacillus subtilis, Bacillus mesentericus Bacillus paralicheniformes,* and combinations thereof.

3. The postbiotic composition for use, according to any of the preceding claims, wherein the bacterial lysates of the genus *Bifidobacterium* are of the species *Bifidobacterium animalis subsp lactis, Bifidobacterium bifidum, Bifidobacterium breve, Bifidobacterium lactis, Bifidobacterium longum, Bifidobacterium infantis, Bifidobacterium animalis,* and combinations thereof.

4. The postbiotic composition for use, according to any of the preceding claims, wherein the bacterial lysates of the genus *Lactobacillus* are of the species that are selected from the group consisting of *Lactobacillus lactis, Lactobacillus acidophilus, Lactobacillus bulgaricus, Lactobacillus casei, Lactobacillus fermentum, Lactobacillus paracasei, Lactobacillus plantarum, Lactobacillus reuteri, Lactobacillus rhamnosus Lactobacillus salivarius, Lactobacillus helvéticus, Lactobacillus gasseri, Lactobacillus johnsonii, Lactobacillus brevis, Lactobacillus kefiri,* and combinations thereof.

5. The postbiotic composition for use, according to any of the preceding claims, wherein the lysates of yeast genus *Saccharomyces* are of the species that are selected from the group consisting of *Saccharomyces cerevisiae, Saccharomyces boulardii,* and combinations thereof.

6. The postbiotic composition for use, according to any of the preceding claims, wherein the bacterial lysates of the genus *Streptococcus* are of the species that are selected from the group consisting of *Streptococcus thermophilus, Streptococcus salivarius,* and combinations thereof.

7. The postbiotic composition for use, according to the preceding claims 1 to 6 wherein the composition comprises lysates of probiotic microorganisms of *Bacillus subtilis, Bacillus coagulans, Bifidobacterium bifidum, Bifidobacterium breve, Bifidobacterium lactis, Bifidobacterium animalis susp lactis, Lactobacillus acidophilus, Lactobacillus casei, Lactobacillus plantarum, Lactobacillus reuteri, Lactobacillus rhamnosus, Saccharomyces cerevisiae* and *Streptococcus thermophilus.*

8. The postbiotic composition for use, according to the preceding claim, wherein the percentage by weight of *Bacillus licheniformis* and *Bacillus subtilis* is approximately the same, and is higher than that of *Bacillus coagulans.*

9. The postbiotic composition for use, according to any of the preceding claims 7 or 8, wherein the percentage by weight of *Bifidobacterium animalis susp lactis* and *Bifidobacterium breve* is approximately the same, and is higher than that of *Bifidobacterium lactis* which is higher than that of *Bifidobacterium bifidum.*

10. The postbiotic composition for use, according to any of the preceding claims 7 to 9, wherein the percentage by weight of *Lactobacillus rhamnosus* and *Lactobacillus acidophilus* is approximately the same, and is smaller than *Lactobacillus casei, Lactobacillus plantarum,* and *Lactobacillus reuteri* which is approximately the same.

11. The postbiotic composition for use, according to claim 1, that comprises lysates of probiotic microorganisms in a percentage amount by weight with respect to the total weight of the lysates of microorganisms of the composition:

    - 0.1% to 10% of bacterial lysates of the genus *Bacillus;*
    - 8% to 40% of bacterial lysates of the genus *Bifidobacterium;*
    - 8% to 60% of bacterial lysates of the genus *Lactobacillus;*
    - 15% to 60% of yeast lysates of the genus *Saccharomyces;*
    - 0.5% to 15% of bacterial lysates of the genus *Streptococcus.*

12. The postbiotic composition for use, according to the preceding claim, that comprises lysates of probiotic microorganisms in a percentage amount by weight with respect to the total weight of the lysates of microorganisms of the composition:

    - 0.1% to 10% of bacterial lysates of *Bacillus licheniformis, Bacillus coagulans* and *Bacillus subtilis;*
    - 8% to 40% of bacterial lysates of *Bifidobacterium animalis susp lactis, Bifidobacterium breve, Bifidobacterium lactis* and *Bifidobacterium bifidum,*
    - 8% to 40% of bacterial lysates of *Lactobacillus acidophilus, Lactobacillus casei, Lactobacillus plantarum, Lactobacillus reuteri* and *Lactobacillus rhamnosus;*
    - 20% to 60% of yeast lysates of *Saccharomyces cerevisiae;*
    - 0.5% to 15% of bacterial lysates of *Streptococcus thermophilus.*

13. A pharmaceutical formulation comprising an effective pharmaceutical amount of the composition according to any of the claims 1 to 12, and at least one pharmaceutically acceptable excipient for use in the treatment of dyslipidemia in a subject.

14. A food supplement comprising the composition of the invention for use in the treatment of dyslipidemia in a subject.

15. The composition for use, according to any of the claims 1 to 12, the pharmaceutical formulation for use, according to claim 13 and/or the food supplement for use, according to claim 14, wherein the dyslipidemia is hypercholesterolemia.

Figure 1

Figure 2

## srebf1

Figure 3

## fasn

Figure 4

## hmgcr

Figure 5

## pparab

Figure 6

| | Europäisches Patentamt<br>European Patent Office<br>Office européen des brevets | **EUROPEAN SEARCH REPORT** | Application Number<br>**EP 23 38 2961** |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | ES 2 783 885 A1 (IGEN BIOLAB GROUP AG [CH]) 18 September 2020 (2020-09-18)<br>* the whole document * | 1-15 | INV.<br>A61K35/66<br>A61K35/742<br>A61K35/744<br>A61K35/745<br>A61K35/747<br>A61K36/064<br>A61P3/06 |
| E | WO 2023/180805 A1 (IGEN BIOLAB GROUP AG [CH]) 28 September 2023 (2023-09-28)<br>* the whole document * | 1-15 | |
| Y | AU 2019 250 114 A1 (BEATTIE CORP PTY LTD [AU]) 21 May 2020 (2020-05-21)<br>* the whole document *<br>* claims 1-14 *<br>* paragraph [0065]; table a *<br>* paragraphs [0097] - [0101] *<br>* paragraph [0110] *<br>* example 7 * | 1-15 | |
| Y | BOUREBABA YASMINA ET AL: "Postbiotics as potential new therapeutic agents for metabolic disorders management", BIOMEDICINE & PHARMACOTHERAPY, ELSEVIER, FR,<br>vol. 153, 16 June 2022 (2022-06-16), XP087167874,<br>ISSN: 0753-3322, DOI: 10.1016/J.BIOPHA.2022.113138<br>[retrieved on 2022-06-16]<br>* figure 1; table 1 *<br>* the whole document * | 1-15 | |

**TECHNICAL FIELDS SEARCHED (IPC)**

A61K
A61P

-/--

The present search report has been drawn up for all claims

| Place of search | Date of completion of the search | Examiner |
|---|---|---|
| Munich | 21 February 2024 | Fayos, Cécile |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

**EP 23 38 2961**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | PARK SEON-JOO ET AL: "Postbiotics against Obesity: Perception and Overview Based on Pre-Clinical and Clinical Studies", INTERNATIONAL JOURNAL OF MOLECULAR SCIENCES , vol. 24, no. 7 29 March 2023 (2023-03-29), page 6414, XP093111451, Basel, CH ISSN: 1422-0067, DOI: 10.3390/ijms24076414 Retrieved from the Internet: URL:https://www.ncbi.nlm.nih.gov/pmc/articles/PMC10095054/pdf/ijms-24-06414.pdf * figure 1; tables 1, 2 * | 1-15 | |
| Y | NAKAMURA FUTOSHI ET AL: "Fragmented Lactic Acid Bacterial Cells Activate Peroxisome Proliferator-Activated Receptors and Ameliorate Dyslipidemia in Obese Mice", JOURNAL OF AGRICULTURAL AND FOOD CHEMISTRY, vol. 64, no. 12, 17 March 2016 (2016-03-17), pages 2549-2559, XP093111575, US ISSN: 0021-8561, DOI: 10.1021/acs.jafc.5b05827 * the whole document * | 1-15 | |
| Y | CN 113 528 367 A (QINGDAO VLAND BIOTECH INC; QINGDAO VLAND BIOTECH GROUP CO LTD) 22 October 2021 (2021-10-22) * the whole document * | 1-15 | **TECHNICAL FIELDS SEARCHED (IPC)** |

-/--

The present search report has been drawn up for all claims

| Place of search | Date of completion of the search | Examiner |
|---|---|---|
| Munich | 21 February 2024 | Fayos, Cécile |

CATEGORY OF CITED DOCUMENTS

X : particularly relevant if taken alone
Y : particularly relevant if combined with another
  document of the same category
A : technological background
O : non-written disclosure
P : intermediate document

T : theory or principle underlying the invention
E : earlier patent document, but published on, or
  after the filing date
D : document cited in the application
L : document cited for other reasons

....................................................................................
& : member of the same patent family, corresponding
  document

EPO FORM 1503 03.82 (P04C01)

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 03/071883 A1 (CT SPERIMENTALE DEL LATTE S P [IT] ET AL.) 4 September 2003 (2003-09-04) * the whole document * | 1-15 | |

TECHNICAL FIELDS
SEARCHED      (IPC)

The present search report has been drawn up for all claims

| Place of search | Date of completion of the search | Examiner |
|---|---|---|
| Munich | 21 February 2024 | Fayos, Cécile |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 23 38 2961

This annex lists the patent family members relating to the patent documents cited in the above-mentioned European search report.
The members are as contained in the European Patent Office EDP file on
The European Patent Office is in no way liable for these particulars which are merely given for the purpose of information.

21-02-2024

| Patent document cited in search report | | Publication date | Patent family member(s) | | Publication date |
|---|---|---|---|---|---|
| ES 2783885 | A1 | 18-09-2020 | ES | 2783723 A1 | 17-09-2020 |
| | | | ES | 2783885 A1 | 18-09-2020 |
| WO 2023180805 | A1 | 28-09-2023 | ES | 2952438 A1 | 31-10-2023 |
| | | | FR | 3133727 A3 | 29-09-2023 |
| | | | WO | 2023180805 A1 | 28-09-2023 |
| AU 2019250114 | A1 | 21-05-2020 | NONE | | |
| CN 113528367 | A | 22-10-2021 | NONE | | |
| WO 03071883 | A1 | 04-09-2003 | AT | E375094 T1 | 15-10-2007 |
| | | | AU | 2003212265 A1 | 09-09-2003 |
| | | | DE | 60316772 T2 | 17-07-2008 |
| | | | EP | 1478246 A1 | 24-11-2004 |
| | | | ES | 2294266 T3 | 01-04-2008 |
| | | | US | 2006251633 A1 | 09-11-2006 |
| | | | WO | 03071883 A1 | 04-09-2003 |

EPO FORM P0459

For more details about this annex : see Official Journal of the European Patent Office, No. 12/82

**REFERENCES CITED IN THE DESCRIPTION**

**Patent documents cited in the description**

- EP 3230479 B1
- ES P201930242
- ES P201930280